# EUROPEAN PATENT APPLICATION

(11) **EP 2 550 967 A1**
(43) Date of publication of application: **30.01.2013**
(21) Application number: 12189550.2
(22) Date of filing: 11.07.2008
(51) Int. Cl.: A61K 31/495, A61K 31/496, A61K 9/00, A61K 9/20, A61P 37/08

(54) **Compressed chewing gum tablet comprising taste-masking agent**

(30) Priority: 11.07.2007 WO PCT/DK2007/050092
(62) Divisional of application: 08758273.0
(71) Applicant: Fertin Pharma A/S, 7120 Vejle (DK)
(72) Inventor: Andersen, Carsten, 7100 Vejle (DK); Lao, My Ly, 7120 Vejle (DK)
(74) Representative: Østergaard, Steen

(57) **Abstract**

The present invention relates to compressed chewing gum tablets comprising a useful antihistamine. In particular, the present invention is directed to compressed chewing gum tablet, which effectively mask the unpleasant tastes of compound according to formula (I), such as cetirizine, contained therein and methods for preparing such compressed chewing gum tablet.

## Description

### FIELD OF THE INVENTION

The present invention is directed to medicament-containing chewing gum compositions. In particular, the present invention is directed to chewing gum compositions, which effectively mask the unpleasant tastes of compounds according to formula I contained therein and methods for preparing such chewing gum.

### TECHNICAL BACKGROUND OF THE INVENTION

People suffering from hay fever, seasonal allergy, and allergy to other substances (such as dust mites, animal dander, and molds), including runny nose, sneezing, and red, itchy, tearing eyes commonly take medications called antihistamines for symptomatic relief.

It is commonly accepted that one group of useful antihistamines is active compounds such as 2-[4-(diphenylmethyl)-1-piperazine] derivatives having the general formula I: 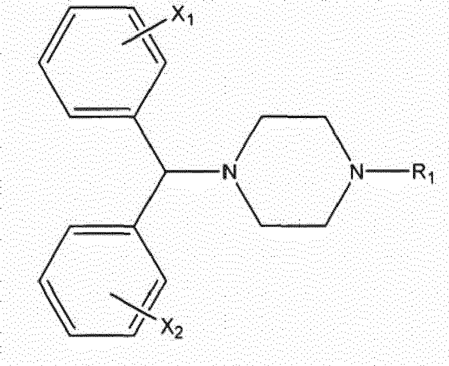 wherein
R₁ is selected from the group consisting of -CH₂CH₂-0-CH₂-R₂, -CH₂CH=CH-Ar₁, -CH₂-Ar₂ and 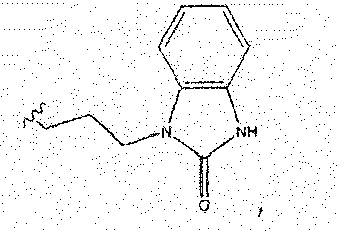
R₂ may be selected from the group consisting of a -CH₂OH group, a -COOH group and a -CONH₂ group;
Ar₁ and Ar₂ are independently an aromatic or heteroaromatic ring with 5 or 6 atoms in the ring, said heteroaromatic ring having 1, 2 or 3 heteroatoms selected from the group consisting of nitrogen, oxygen and sulfur, said ring being unsubstituted or substituted with C₁₋₄ alkyl, preferably methyl or tertiary butyl, Ar₁ and Ar₂ preferably being unsubstituted phenyl or phenyl substituted with C₁₋₄ alkyl, preferably methyl or tertiary butyl; and
X₁ and X₂ may independently be selected from the group consisting of a hydrogen atom, a halogen atom, a straight-chain or branched C₁-C₄ alkoxy group or a trifluoromethyl group; as well as pharmaceutically acceptable salts, geometrical isomers, enantiomers, diastereomers and mixtures thereof.

In the present context, the term "C₁₋₄-alkoxy" is intended to mean C₁₋₄-alkyl-oxy, such as methoxy, ethoxy, n-propoxy, isopropoxy, n-butoxy, isobutoxy, sec-butoxy and tert-butoxy. Furthermore, the term "halogen" includes fluorine, chlorine, bromine and iodine.

One of the most popular and effective antihistamines is cetirizine, which in its dihydrochloride form marketed under the tradename Zyrtec®, the (S)-enantiomer thereof, levocetirizine, in its dihydrochloride form marketed under the trade name Xyzal® and efletirizine in its dihydrochloride form.

This medicament however is very bitter and has a highly unacceptable taste. Accordingly, it is usually administered in syrups where masking of the bitter taste is relatively easy. Cetirizine has also been incorporated in tablets. Such tablets are formulated as white, film-coated, rounded-off rectangular shaped tablets so that its displeasing organoleptic qualities completely bypass the sense of taste.

Attempts to incorporate cetirizine in chewing gums, however, have yielded products, which are generally unacceptable. Some cetirizine-containing chewing gums have been found to have the bitter taste, and unacceptable flavour associated with this agent become especially noticeable after the first two to five minutes of chewing. In other chewing gum products, cetirizine has been coated in waxes in order to combat the unpleasant taste.

For example in US 2006/0038039 an oral pharmaceutical composition is described containing at least two separate formulations: a first formulation, which contains an active compound according to the above formula I and which first formulation does not contain polyols having a molecular weight of less than 300 in a molar ratio between the polyol and active compound of formula I above 10; and a second formulation, which contains one or more polyol(s) with a molecular weight of less than 3000 and is free of any drug.

It is a well-known problem in the chewing gum industry to find suitable agents, which both mask the bitter taste of active compounds and give good palatability to medicated chewing gums. As it has been shown that the above-mentioned taste-masking agents are the most useful agents, there is an industrial need to find a solution to how these agents can be used in medicated chewing gums.

### SUMMARY OF THE INVENTION

Thus, the present invention provides a medical formulation of an active compound according to formula I having a consumer acceptable taste and good palatability. Accordingly, in a first aspect, the present invention relates to a compressed chewing gum tablet comprising at least one active compound selected from 2-[4-(diphenylmethyl)-1-piperazine] derivatives having the general formula I: 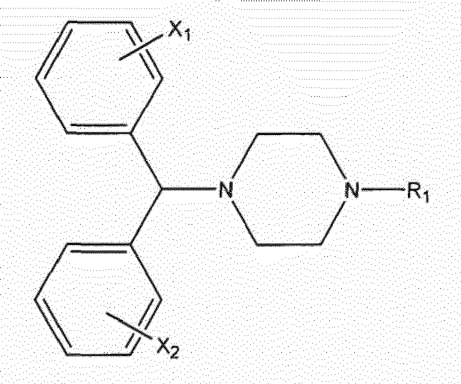 wherein
R₁ is selected from the group consisting of -CH₂CH₂-O-CH₂-R₂, -CH₂CH=CH-Ar₁, -CH₂-Ar₂ and 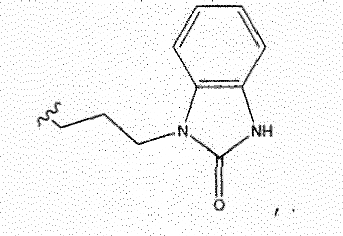
R₂ may be selected from the group consisting of a -CH₂OH group, a -COOH group and a -CONH₂ group;
Ar₁ and Ar₂ are independently an aromatic or heteroaromatic ring with 5 or 6 atoms in the ring, said heteroaromatic ring having 1, 2 or 3 heteroatoms selected from the group consisting of nitrogen, oxygen and sulfur, said ring being unsubstituted or substituted with C₁₋₄ alkyl, preferably methyl or tertiary butyl, Ar₁ and Ar₂ preferably being unsubstituted phenyl or phenyl substituted with C₁₋₄ alkyl, preferably methyl or tertiary butyl; and
X₁ and X₂ may independently be selected from the group consisting of a hydrogen atom, a halogen atom, a straight-chain or branched C₁-C₄ alkoxy group or a trifluoromethyl group; as well as pharmaceutically acceptable salts, geometrical isomers, enantiomers, diastereomers and mixtures thereof;
a taste-masking agent and a first compressed module comprising compressed chewing gum particles containing gum base; and wherein the weight ratio between the taste-masking agent and the compound according to formula I is at least 5:1.

Without being bound theory, the inventors suggest that the obtained good taste and palatability of the present chewing gum tablet is achieved by providing a well-balanced ratio between the active compound according to formula I and the taste-masking agent in combination with the use of compressed particles containing gum base. This combination results in an effective masking of the bitter taste of the active compound, as well as an acceptable texture and an interesting taste experience.

It has further been found that when the active compound and the taste-masking agent are positioned as described in detailed below, the medicament and the agent are co-released upon chewing resulting in an effective masking of the medicaments bitter taste.

In a further aspect of the present invention there is provided a method of preparing a compressed chewing gum tablet according to the invention comprising one compressed module, the method comprising the steps of: a) providing a portion comprising an active compound according to formula I, a portion comprising taste-masking agent, and chewing gum particles containing gum base; b) optionally providing one or more further chewing gum ingredients; c) dosing the portion comprising the compound according to formula I, the portion comprising taste-masking agent, and the chewing gum particles containing gum and optionally the one or more further chewing gum ingredients; and d) compressing a) and b) after dosing, to obtain a first compressed module.

In a still further aspect, the present invention provides a method of preparing a compressed chewing gum tablet according to the invention comprising one compressed module, the method comprising the steps of: a) providing a portion comprising an active compound according to formula I, a portion comprising taste-masking agent, and chewing gum particles containing gum base; b) optionally providing one or more further chewing gum ingredients; c) mixing the portion comprising the compound according to formula **I,** the portion comprising taste-masking agent, and the chewing gum particles containing gum base, and optionally the one or more further chewing gum ingredients, thus obtaining a mixture; and d) compressing the mixture to obtain a first compressed module.

Yet further, the present invention provides a method of preparing a compressed chewing gum tablet according to the invention comprising two compressed modules, the method comprising the steps of: a) providing chewing gum particles containing gum base and optionally portion(s) comprising one or more chewing gum ingredients; b) providing a portion comprising an active compound according to formula I and a portion comprising a taste-masking agent; c) compressing a) to obtain a first compressed module; d) contacting the first compressed module with b); and e) compressing b) and the first compressed module, to obtain a coherent compressed chewing gum tablet comprising a first compressed module and a second compressed module.

In a still further aspect, there is provided a method of preparing a compressed chewing gum tablet according to the invention comprising two compressed modules, the method comprising the steps of: a) providing chewing gum particles containing gum base and a portion comprising an active compound according to formula I, and optionally portion(s) comprising one or more chewing gum ingredients; b) providing a portion comprising taste-masking agent; c) compressing a) to obtain a first compressed module; d) contacting the first compressed module with b); and e) compressing b) and the first compressed module, to obtain a coherent compressed chewing gum tablet comprising a first compressed module and a second compressed module.

A further aspect relates to a method of preparing a compressed chewing gum tablet according to the invention comprising three compressed modules, the method comprising the steps of: a) providing chewing gum particles containing gum base, a portion comprising a taste-masking agent, and optionally portion(s) comprising one or more chewing gum ingredients; b) providing a portion comprising tablet material and optionally a portion comprising an active compound according to formula I; c) providing a portion comprising tablet material and a portion comprising an active compound according to formula I; and d) locating b) and c) on opposite sites of a) following a sequence of one or more compressing step(s), to obtain a coherent compressed chewing gum tablet comprising a first compressed module and a second compressed module and a third compressed module,

In a further aspect, there is provided a method of preparing a compressed chewing gum tablet according to the invention comprising three compressed modules, the method comprising the steps of: a) providing chewing gum particles containing gum base, a portion comprising an active compound according to formula I, and optionally portion(s) comprising one or more chewing gum ingredients; b) providing a portion comprising tablet material and optionally a portion comprising a taste-masking agent; c) providing a portion comprising tablet material and a portion comprising a taste-masking agent; and d) locating b) and c) on opposite sites of a) following a sequence of one or more compressing step(s), to obtain a coherent compressed chewing gum tablet comprising a first compressed module and a second compressed module and a third compressed module.

A final aspect relates to a method of preparing a compressed chewing gum tablet according to the invention comprising three compressed modules, the method comprising the steps of a) providing chewing gum particles containing gum base, and optionally portion(s) comprising one or more chewing gum ingredients; b) providing a portion comprising tablet material and a portion comprising an active compound according to formula I and a portion comprising a taste-masking agent; c) providing a portion comprising tablet material and a portion comprising an active compound according to formula I and a portion comprising a taste-masking agent; and d) locating b) and c) on opposite sites of a) following a sequence of one or more compressing step(s), to obtain a coherent compressed chewing gum tablet comprising a first compressed module and a second compressed module and a third compressed module.

### BRIEF DESCRIPTION OF THE FIGURES

The invention will now be described with reference to the drawings of which
Figs. 1a-1b illustrate a two-layer compressed tablet according to an embodiment of the invention,
Figs. 2a-2b illustrate a three layer compressed tablet according to an embodiment of the invention,
Figs. 3a-3b illustrate a further two layer compressed tablet according to an embodiment of the invention,
Figs. 4a-4b illustrate a further two layer compressed tablet according to an embodiment of the invention, and where
Figs. 5a-5b illustrate a further two layer compressed tablet according to an embodiment of the invention.

### DETAILED DESCRIPTION OF THE INTENTION

### Definitions

In the present context, the expression "taste-masking agent" relates to one or more agents or compounds which, optionally together, successfully mask or cover the bitter taste of the compound according to formula I, but which simultaneously provide the chewing gum a good palatability. In a preferred embodiment, the taste-masking agent comprises a polyol sweetener.

In the present context, the term "gum base" refers in general to a commercially available gum base suitable for production of chewing gum. Such gum bases normally comprise one or more elastomeric compounds which may be of synthetic or natural origin, one or more resinous compounds which may be of synthetic or natural origin and softening compounds.

The term "gum base composition" as used herein may be a gum base as defined above comprising one or more ingredients (e.g. sweetener, flavour, colouring agents, fillers etc.) as described below.

The term "chewing gum composition" is the final formulation, which constitutes at least a part of the compressed chewing gum tablets ready for sale or use by the consumer. A chewing gum composition may comprise an active compound according to formula I, a taste masking agent, sweetener and/or flavour and optionally other ingredients like colouring agents, enzymes, humectants, flavour enhancers, anticaking agents etc,

Furthermore, the expression "compressed chewing gum tablets" denote a ready for use chewing gum tablet, e.g. comprising compressed particles containing gum base possibly mixed with an active compound according to formula I, a taste-masking agent, sweeteners, flavour or other ingredients and optionally coated. As described in detail below, a compressed chewing gum tablet is produced by an initial conventional mixing of the gum base with e.g. water-insoluble ingredients such as elastomers and resins, followed by a granulation or the like of the obtained gum base mix. The obtained particles containing gum base may then be mixed with further chewing gum ingredients, such as an active compound according to formula I, a taste-masking agent, sweeteners and flavours. The final mix may then be compressed under high pressure (typically when applying cooling) into to a compressed chewing gum tablet or a compressed module.

Thus, the expression "chewing gum particles containing gum base" refers to particulated material of chewing gum composition and is to be understood as any form of chewing gum particles containing a certain amount of gum base as described in detail below. The chewing gum particles may be in any suitable form such as pellets, granules, agglomerates, powder. Thus, in some embodiments, the particles have been particulated prior to application, Particulation may be in any form of "building up" particles from smaller primary particles into macro particles or in any form of "building down" from larger substances into macro particles. Any form of particulation may be applied, such as granulation, pelletizing, agglomeration, or any other suitable means for particulation, as described below. Thus, the particles may also to be understood as macroparticles.

Furthermore, the "compressed chewing gum particles containing gum base" refers to a portion of chewing gum particles which become compressed after mixed with e.g. an active compound according to formula I, a taste-masking agent, sweeteners or flavours.

### Preferred embodiments

The present invention relates to a medicated chewing gum tablet with an effective amount of an active compound according to formula I, having a costumer acceptable taste during all chewing phases.

This is achieved by preferably placing the compound according to formula I and a taste-masking agent in the chewing gum in such a way that, upon chewing, some of the taste-masking agent releases when the compound releases. The inventors of the present invention found, that such a co-release of the compound according to formula I and the taste-masking agent results in an effective masking of the bitter taste of the compound. This masking effect does not depend on the release of the whole portion of taste-masking agent. Thus, under some circumstances, the compound and the taste-masking agent are positioned in such a way that, upon chewing, not all taste-masking agent releases during the release of the compound according to formula I.

Accordingly, the present invention provides a compressed chewing gum tablet comprising at least one active compound selected from 2-[4-(diphenylmethyl)-1-piperazine] derivatives having the general formula I: 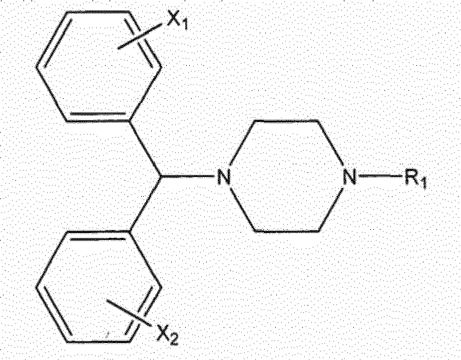 wherein
R₁ is selected from the group consisting of -CH₂CH₂-O-CH₂-R₂, -CH₂CH=CH-Ar₁, -CH₂-Ar₂ and 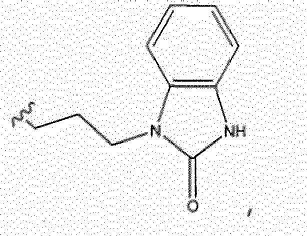
R₂ may be selected from the group consisting of a -CH₂OH group, a -COOH group and a -CONH₂ group;
Ar₁ and Ar₂ are independently an aromatic or heteroaromatic ring with 5 or 6 atoms in the ring, said heteroaromatic ring having 1, 2 or 3 heteroatoms selected from the group consisting of nitrogen, oxygen and sulfur, said ring being unsubstituted or substituted with C₁₋₄ alkyl, preferably methyl or tertiary butyl, Ar₁ and Ar₂ preferably being unsubstituted phenyl or phenyl substituted with C₁₋₄ alkyl, preferably methyl or tertiary butyl, and
X₁ and X₂ may independently be selected from the group consisting of a hydrogen atom, a halogen atom, a straight-chain or branched C₁-C₄ alkoxy group or a trifluoromethyl group; as well as pharmaceutically acceptable salts, geometrical isomers, enantiomers, diastereomers and mixtures thereof;
a taste-masking agent and a first compressed module comprising compressed chewing gum particles containing gum base, wherein the weight ratio between the taste-masking agent and the compound according to formula I is at least 1:5, and preferably at least 5:1.

### The taste-masking agent

As defined above, the taste-masking agents are one or more agents or compounds which, optionally together, successful mask or cover the bitter taste of the compound according to formula I, but which simultaneously provide the chewing gum a good palatability. In a preferred embodiment, the taste-masking agent comprises a polyol sweetener.

In useful embodiments, the polyol sweetener is a sugar which may be selected from the group consisting of dextrose, sucrose, maltose, fructose and lactose.

In another preferred embodiment, the chewing gum comprises a polyol sweetener, which is a sugar alcohol. A useful sugar alcohol may be selected from the group consisting of xylitol, sorbitol, mannitol, maltitol, isomaltol or isomalt, erythritol, lactitol, maltodextrin and hydrogenated starch hydrolysates.

The taste masking agent, when comprising a polyol sweetener, may be used in an amount in the range of 90-100% by weight of the taste masking agent, preferably in the range 95-99.9% and even more preferred in the range 97-99.5% by weight of the taste masking agent.

The presence of mannitol in the compressed chewing gum tablet surprisingly appears to improve the stability of the active compound according to formula I relative to chewing gum tablets containing other low molecular weight polyol sweeteners such as sorbitol, Thus, in a preferred embodiment of the invention, the polyol sweetener of the compressed chewing gum tablet comprises mannitol in an amount of at least 25% by weight of the total amount of polyol sweetener of the compressed chewing gum tablet, such as in an amount of at least 50% by weight, preferably in an amount of at least 75%, and even more preferred in an amount of at least 95% by weight of the total amount of polyol sweetener of the compressed chewing gum tablet. In an embodiment of the invention, substantially all polyol sweetener of the compressed chewing gum tablet is mannitol.

In another embodiment of the invention, the polyol sweetener of the second compressed module comprises mannitol in an amount of at least 25% by weight of the total amount of polyol sweetener of the second compressed module, such as in an amount of at least 50% by weight, preferably in an amount of at least 75%, and even more preferred in an amount of at least 95% by weight of the total amount of polyol sweetener of the second compressed module.

In an embodiment of the invention, substantially all polyol sweetener of the second compressed module is mannitol.

In an interesting embodiment, the chewing gum according to the invention is one wherein the taste-masking agent comprising a high intensity sweetener or a flavour. Useful high intensity sweetener may be selected from the group consisting of sucralose, neotame, aspartame, salts of acesulfame, alitame, saccharin and its salts, cyclamic acid and its salts, glycyrrhizin, dihydrochalcones e.g. NHDC, thaumatin, monellin, stevioside, Twinsweet (aspartame-acesulfame salt) and combinations thereof.

Such a high intensity sweetener may be used in the chewing gum according to the invention in an amount in the range of 0.01-5% by weight of the taste masking agent, preferably in the range 0.1-2% and even more preferred in the range 0.4-1.5% by weight of the taste masking agent.

In an interesting embodiment, the taste-masking agent comprises one or more high intensity sweetener in an amount in the range of 0.01-5% by weight of the taste-masking agent and one or more polyol sweetener in an amount in the range of 90-100% by weight of the taste masking agent, and preferably the taste-masking agent comprises high intensity sweetener in an amount in the range of 0.1-2% by weight of the taste-masking agent and polyol sweetener in an amount in the range of 98-99.9% by weight of the taste masking agent.

Under some circumstances it may be desirable to use more than one taste-masking agent, Thus, in a useful embodiment, the chewing gum tablet according to the invention is one, which comprises an additional taste-masking agent and may, optionally, be located between the compressed chewing gum particles containing gum base. Such additional taste-masking agent may be selected from the group consisting of a salt of gluconate, such as e.g. sodium gluconate.

### Compound according to formula I

Active compounds according to formula I may be added at any time during the process of preparing the chewing gum. However, it is presently preferred that the active compounds are added to the chewing gum subsequent to any significant heating or mixing. In other words, the active compounds should preferably be added immediately prior to the compression of the final tablet. Referring to the process described below, the adding of active compounds may be cautiously blended with pre-mixed gum base particles and further desired ingredients, immediately prior to the final compression of the tablet.

The active compounds according to formula I are preferably orally active and selective histamine Hi-receptor antagonists. In preferred embodiments of the invention, the active compound according to formula I is so selected that X₁ is a hydrogen atom, X₂ is a halogen atom, and R₁ is -CH₂CH₂-0-CH₂-COOH. Preferably, the active compound according to formula I is a salt, e.g. a dihydrochloride salt.

In another embodiment of the invention, the active compound according to formula I is selected from the group consisting of buclizine, cetirizine, chlorcyclizine, cinnarizine, cyclizine, hydroxyzine, levocetirizine, meclozine, efletirizine and oxatomide, as well as any pharmaceutically acceptable salts, geometrical isomers, enantiomers, diastereomers and mixtures thereof.

Useful active compounds according to formula I may be selected from the group consisting of is cetirizine, the dihydrochloride salt of cetirizine, levocetirizine, the dihydrochloride salt of levocetirizine, efletirizine, and the dihydrochloride salt of eftetirizine.

In a preferred embodiment, the active compound according to formula I is cetirizine, i.e. 2-[2-[4-[(4-chtorophenyl)-phenyl-methyl] piperazin-1-yl]ethoxy]acetic acid having the following formula II: 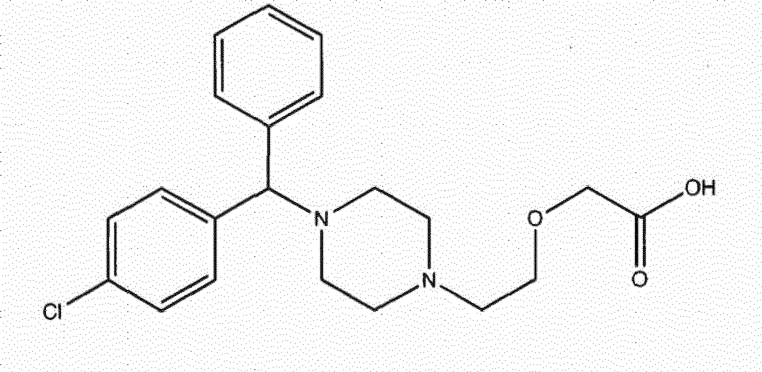 or a salt thereof, preferably cetirizine dihydrochloride.

It has been found, that a well-balanced ratio between cetirizine and a taste-masking agent in combination with the use of compressed particles containing gum base results in a pleasant taste experience and excellent texture of the chewing gum tablet, as well as an effective masking of the bitter taste of cetirizine.

In embodiments of the invention, the chewing gum comprises the compound according to formula I, e.g. cetirizine or cetirizine dihydrochloride, in an amount in the range of 0.1 - 50 mg, preferably in the range of 1 - 30 mg, and even more preferably in the range of 5-25 mg.

As will be apparent to the person skilled in the art, the active compound according to formula I can be used for the present invention in any suitable form, which includes encapsulates, complexes, or clathrates of the active compound according to formula I.

### Different types of chewing gum tablets

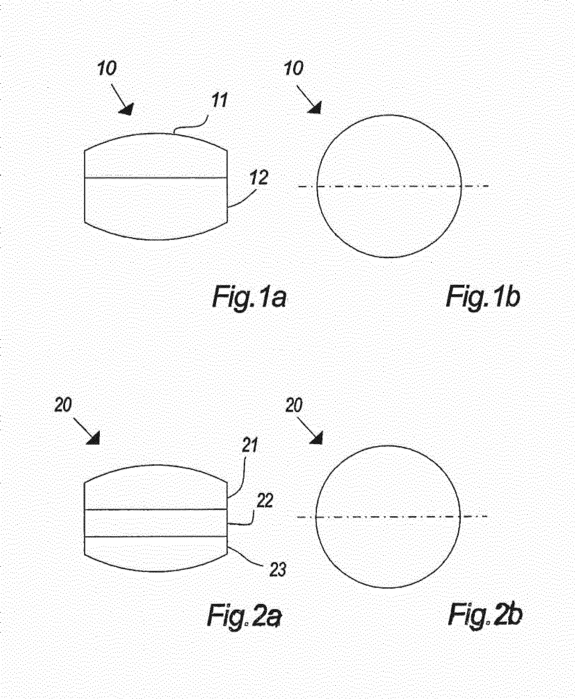

In an embodiment of the invention, the compressed chewing gum tablet is one wherein the first compressed module is on top of a second compressed module. Figure 1a and 1b illustrates such a compressed chewing gum tablet according to the invention. The illustrated chewing gum tablet 10 comprises two chewing gum modules 11 and 12.

According to the illustrated embodiment, each module is simply comprised by a layer. The multi-module tablet may in this embodiment be regarded as a two-layer or *two-module* chewing gum tablet 10. The two modules 11 12 are adhered to each other. Different processes may be applied for the purpose as described below. However, according to a preferred embodiment of the invention, the mutual adhering between the two modules is obtained by the compression of one module 11 onto the other module 12.

The illustrated chewing gum tablet 10 may for example comprise a non-gum base-containing module 11 and a gum base-containing module 12. Thus, in an embodiment of the invention, the compressed chewing gum tablet in one wherein the second compressed module, i.e. the module 11, comprises compressed tablet material. Examples of useful tablet material are described below.

However, it may under some circumstances be useful also to include gum base in the second compressed module. Thus, in a useful embodiment, the second compressed module, i.e. the module 11, comprises compressed chewing gum particles containing gum base and optionally one or more further chewing gum ingredients.

Figure 2a illustrates a cross-section of a compressed chewing gum tablet according to the invention and figure 2b illustrates the chewing gum from above. Thus, an embodiment of the present invention is one wherein the first compressed module is on top of a second compressed module on top of a third compressed module, or described in another manner, the first module is located between two outer modules.

The illustrated chewing gum tablet 20 in figure 2a may in one embodiment comprises a three-module chewing gum of which the lowest module or third module 23 comprises compressed tablet material and the modules 21 and 22 are as described above in figure 1. In a further embodiment, the compressed chewing gum tablet 20 is one wherein the third compressed module 23 comprises compressed chewing gum particles containing gum base and optionally one or more further chewing gum ingredients. The modules 21 and 22 may be as described above in Figure 1.

However, under some circumstances it may be useful to provide a chewing gum tablet having three modules comprising compressed chewing gum particles containing gum base. Thus, in a useful embodiment, the compressed chewing gum tablet is one wherein said first, and/or third compressed module comprises compressed chewing gum particles containing gum base and one or more further chewing gum ingredients.

An interesting embodiment of the invention is where the chewing gum tablet 20 comprises three modules, wherein the first compressed module 22 comprises compressed chewing gum particles containing gum base and one or more further chewing gum ingredients, and where said module is located between two compressed outer modules 21 and 23 comprising compressed tablet material.

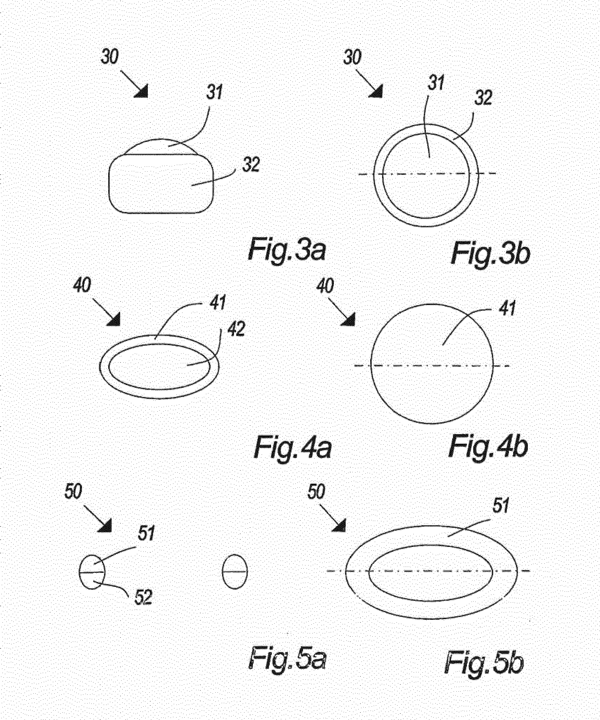

Figure 3a illustrates a cross-section of a compressed chewing gum tablet 30 according the invention and illustrated in figure 3b from above. The illustrated chewing gum tablet 30 comprises a module 32 comprising compressed chewing gum particles containing gum base and optionally one or more further chewing gum ingredients upon which a second module 31 is arranged. The module 31 may comprise compressed tablet material or compressed chewing gum particles containing gum base and one or more further chewing gum ingredients.

Figure 4a cross-section of a further compressed multi-modular chewing gum tablet 40 according to the invention and illustrated in figure 4b from above. The tablet 40 differs somewhat from the other described tablets in the sense that the tablet comprises a module 42 comprising compressed chewing gum particles containing gum base and optionally one or more further chewing gum ingredients forming a gum centre. The module 42 is encapsulated by a surrounding module 41. The module 41 may comprise compressed tablet material or compressed chewing gum particles containing gum base and one or more further chewing gum ingredients.

Figure 5a illustrates a cross-section of a compressed multi-modular chewing gum tablet 50 according to the invention and illustrated in figure 5b from above. According to the frustrated embodiment, showing a ring-formed two-module tablet 50, a module 52 comprising compressed chewing gum particles containing gum base a certain concentration and optionally one or more further chewing gum ingredients whereas the other module 51 comprises compressed tablet material.

Alternatively, the chewing gum module 51 may comprise a content of compressed chewing gum particles containing gum base differing from that of the content of module 52, thereby facilitating a chewing gum providing at least two different release profiles in one piece.

The compressed chewing gum tablet of the present invention comprises an active compound according to formula I as well as a taste-masking agent. An advantage of the compressed chewing gum tablet according the present invention is that the compound according to formula I is released faster than from conventionally mixed chewing gum. An additional advantage is that the specific location of the compound according to formula I and the taste-masking agent provides for a co-release of the compound according to formula I and the taste-masking agent. In this context, the "co-release" means that when the compound according to formula I is released from the chewing gum during chewing, some taste-masking agent is also released. A preferred embodiment of the invention is one, when the compound according to formula I is released from the chewing gum during chewing, an amount of taste-masking agent sufficient to mask the bitter taste of the compound according to formula I is also released.

### Active compound according to formula I between the particles

In a preferred embodiment, the chewing gum of the present invention, is one wherein the compound according to formula I and the taste-masking agent are located between the compressed chewing gum particles containing gum of the first compressed module, and/or, if a second compressed module is present, between the compressed chewing gum particles containing gum base of the second compressed module. In a useful embodiment, the compound according to formula I and the taste-masking agent are located in the compressed chewing gum particles containing gum base of the first and/or second compressed module.

Without being bound by theory, the inventors suggest that the effective masking effect results from a quick release and/or co-release of the taste-masking agent in the chewing gum. This is achieved by placing the compound according to formula I together or in direct contact with the taste-masking agent so that both compounds releases simultaneously.

### Compound according to formula I located in different compressed modules

A quick release of the taste-masking agent and/or co-release of the taste-masking and the compound according to formula I in the chewing gum may be obtained by placing the agent and the compound together or separate in different compressed modules of the chewing gum according to the invention.

Thus, in a useful embodiment, the chewing gum of the present invention is one comprising a first and a second compressed module, wherein the first compressed module comprises compressed chewing gum particles containing gum base, an active compound according to formula I, and a taste-masking agent.

It is well known for a skilled person in the art to prepare a chewing gum with multiple compressed modules. In accordance with invention, the second compressed module may preferable not comprise gum base, or only comprise at most 1% gum base, such as at the most 0.5% gum base by weight of the second compressed module.

In a further preferred embodiment, the compressed chewing gum tablet is one comprising a first and a second compressed module, wherein the first compressed module comprises compressed chewing gum particles containing gum base, and an active compound according to formula I, and the second compressed module comprises a taste-masking agent.

In an even further preferred embodiment, the compressed chewing gum tablet is one comprising a first and a second compressed module, wherein the first compressed module comprises compressed chewing gum particles containing gum base, and a taste-masking agent, and the second compressed module comprises an active compound according to formula I.

A quick release of the taste-masking agent and/or co-release of the taste-masking agent and the compound according to formula I in the chewing gum may also be obtained by placing the two compounds together in a different compressed module of the chewing gum than the module comprising compressed chewing gum particles containing gum base.

Thus, in a still further preferred embodiment, the compressed chewing gum tablet is one comprising a first and a second compressed module, wherein the first compressed module comprises compressed chewing gum particles containing gum base, and the second compressed module comprises an active compound according to formula I and a taste-masking agent.

In an embodiment, the compressed chewing gum tablet comprises a first, a second and a third compressed module, wherein the first compressed module comprises compressed chewing gum particles containing gum base, and the second compressed module may comprise an active compound according to formula I and the third compressed module may comprise a taste-masking agent. In a further embodiment, the compressed chewing gum tablet is one, wherein the first compressed module comprises further a taste-masking agent and the compressed modules comprising compressed tablet material comprises an active compound according to formula I.

In accordance with the invention, the compound according to formula I and the taste-masking agent are compressed together with the chewing gum particles containing gum base. However, this process is described in detail below.

Although it is under some circumstances preferred that the second compressed module not comprises gum base, or only comprise at most 1% gum base, such as at the most 0.5% gum base by weight of the second compressed module, it may be useful to incorporate compressed chewing gum particles containing gum base in the second module of the chewing gum.

### Ratio between active compound to Formula I and taste-masking agent

In accordance with the present invention, the ratio between the compound according to formula I and the taste-masking agent is well-balanced as it is presently believed that a specifically defined ratio is one of the reasons for providing a good taste and palatability of the present compressed chewing gum tablet.

In a preferred embodiment, the weight ratio between the taste-masking agent and the compound according to formula I is at least 5: 1, preferably at least 10: 1, such as at least 20:1, and even more preferred at least 50:1, such as at least 100:1.

In a useful embodiment, the chewing gum is one wherein the weight ratio between the taste-masking agent and the compound according to formula I is in the range of 5:1 - 500:1, preferably in the range of 10:1 - 250:1, such as in the range of 20:1 - 200:1, and even more preferred in the range of 40:1 - 175:1, such as in the range of 50:1 - 150:1.

In a further preferred embodiment, the weight ratio between the taste-masking agent of the first compressed module and the compound according to formula of the first compressed module is at least 5:1, preferably at least 10:1, such as at least 20:1, and more preferred at least 50:1, such as at least 100:1.

In a useful embodiment, the chewing gum is one wherein the weight ratio between the taste-masking agent of the first compressed module and the compound according to formula I of the first compressed module is in the range of 5:1 - 500:1, preferably in the range of 10:1 - 250:1, such as in the range of 20:1 - 200:1, and even more preferred in the range of 40:1 - 175:1 such as in the range of 50:1 - 150:1.

In another preferred embodiment, the weight ratio between the taste-masking agent of the second compressed module and the compound according to formula I of the first compressed module is at least 5:1, preferably at least 10:1, such as at least 20:1, and even more preferred at least 50:1, such as at least 100:1.

In a useful embodiment, the chewing gum is one wherein the weight ratio between the taste-masking agent of the second compressed module and the compound according to formula I of the first compressed module is in the range of 5:1 - 500:1, preferably in the range of 10:1 - 250:1, such as in the range of 20:1 - 200:1, and even more preferred in the range of 40:1 - 175:1, such as in the range of 50:1 - 150:1.

In another preferred embodiment, the weight ratio between the taste-masking agent and of the first compressed module and the compound according to formula I of the second compressed module is at least 5:1, preferably at least 10:1, such as at least 20:1, and even more preferred at least 50:1, such as at least 100:1

In a useful embodiment, the chewing gum is one wherein the weight ratio between the taste-masking agent of the first compressed module and the compound according to formula I of the second compressed module is in the range of 5:1 - 500:1, preferably in the range of 10:1 - 250:1, such as in the range of 20:1 - 200:1, and even more preferred in the range of 40:1 - 175:1, such as in the range of 50:1 - 150:1.

In a further preferred embodiment, the weight ratio between the taste-masking agent of the second compressed module and the compound according to formula I of the second compressed module and is at least 5: 1, preferably at least 10: 1, such as at least 20: 1, and even more preferred at least 50: 1, such as at least 100:1.

In a useful embodiment, the chewing gum is one wherein the weight ratio between the taste-masking agent of the second compressed module and the compound according to formula I of the second compressed module is in the range of 5:1 - 500:1, preferably in the range of 10:1 - 250:1, such as in the range of 20:1 - 200:1, and even more preferred in the range of 40:1 - 175:1, such as in the range of 50:1 - 150:1.

In a preferred embodiment, the chewing gum tablet comprising a first, second and third module, the weight ratio between the taste-masking agent of the third compressed module and the compound according to formula I of the second compressed module is at least 5: 1, preferably at least 10: 1, such as at least 20: 1, and even more preferred at least 50:1, such as at least 100:1.

In a useful embodiment, the chewing gum is one wherein the weight ratio between the taste-masking agent of the third compressed module and the compound according to formula I of the second compressed module is in the range of 5:1 500:1, preferably in the range of 10:1 - 250:1, such as in the range of 20:1 - 200:1, and even more preferred in the range of 40:1 - 175:1, such as in the range of 50:1 - 150:1.

In another preferred embodiment, the weight ratio between the taste-masking agent of the first compressed module and the compound according to formula I of the modules comprising tablet material and is at least 5: 1, preferably at least 10:1, such as at least 20: 1, and even more preferred at least 50: 1, such as at least 100. 1.

In a useful embodiment, the chewing gum is one wherein the weight ratio between the taste-masking agent of the first compressed module and the compound to formula I of the modules comprising tablet material is in the range of 5:1 - 500:1, preferably in the range of 10:1 - 250:1, such as in the range of 20:1 - 200:1, and even more preferred in the range of 40:1 - 175 1, such as in the range of 50:1 - 150:1.

### The chewing gum particles containing gum base

The gum base contained in the compressed modules of the chewing gum tablet according to the invention is typically present in the form of compressed gum base particles. The manufacturing of gum base particles is described below. However, the particles may be manufactured according to conventional methods or e.g. those described in the EP1474993, EP1474994 and EP1474995, hereby incorporated by reference.

It was found, that by using compressed particles of gum base in combination with a specifically defined ratio between the active compound according to formula I and the taste-masking agent, as described above, results in an acceptable texture and an interesting taste experience as well as an effective masking of the bitter taste of the active compound.

In accordance with the invention, the chewing gum particles comprise gum base. As described above, the chewing gum particles may have any form of chewing gum particles containing a certain amount of gum base. The content of gum base in the particles may vary. In some embodiments, the amount of gum base in the chewing gum particles is rather high, such in the range of 40-99% by weight of the chewing gum particles. In some embodiments, the amount of gum in the chewing gum particles is in the range of 40-90% by weight of the chewing gum particles, such as in the range of 40-80% by weight, including in the range of 40-70% by weight, e.g. in the range of 40-50% by weight, such as in the range of 50-85% by weight, including in the range of 50-75% by weight, e.g. in the range of 50-55% by weight of the chewing gum particles.

In some other embodiments, the amount of gum base in the chewing gum particles is lower, such as in the range of 15-60% by weight of the chewing gum particles. Other useful amounts may vary in the range of 20-60% by weight of the chewing gum particles, such as in the range of 20-50%, including in the range of 20-40% by weight, e.g. in the range of 30-55% by weight, such as in the range of 30-45% by weight of the chewing gum particles. The remaining content of the chewing gum particles may comprise one or more of the below chewing gum ingredients.

In some embodiments, the particles are made entirety of gum base, substantially without conventional chewing gum ingredients. In this case, the chewing gum ingredients may be applied in the compression process, such as by adding the chewing gum ingredients together with the gum base particles for compression.

In some other embodiments, the particles are made of chewing gum, substantially without further needs for chewing gum ingredients in the compression process. Of course, intermediate solutions may be applicable, such as a varying amount of chewing gum ingredients in the chewing gum particles or in the compression process.

It may be preferred to apply at least a certain amount of high intensity sweetener and/or flavour and/or colour to the chewing gum particles in some embodiments of the invention, such as in case the chewing gum particles substantially consist of gum base.

In preferred embodiments, the average particle size of the particles is in the range of 50-2000 micrometer measured as the longest dimension of the particle, preferably in the range of 100-1500 micrometer, and even more preferred in the range of 200-1300 micrometer.

In even more preferred embodiments, the chewing gum tablet is one wherein at least 70% of the particles have a particle size in the range of 50-2000 micrometer measured as the longest dimension of the particle, preferably in the range of 100-1500 micrometer, and even more preferred in the range of 200-1300 micrometer.

### Gum base

In a preferred embodiment, the chewing gum composition comprises a gum base. Typically, a useful gum base compositions typically comprise one or more elastomeric compounds which may be of synthetic or natural origin, one or more resinous compounds which may be of synthetic or natural origin, fillers, softening compounds and minor amounts of miscellaneous ingredients such as antioxidants and colorants, etc. One advantage of the present invention is that there is no need to adjust the content of other chewing gum ingredients in order to maintain the desired texture. Furthermore, a very interesting observation is that no disintegration of the chewing gum occurs upon chewing,

The compressed module containing gum base according to the invention may typically be made on the basis of gum base particles. The gum base particles are made on the basis of a gum base. In addition to the above definition of the expression "gum base", the expression further refers to the water-insoluble part of the chewing gum tablet which typically constitutes 10 to 99% by weight including the range of 20-99% by weight of the total chewing gum composition, such as the range of 30-99% by weight of the total chewing gum composition. In preferred embodiments, the chewing gum composition comprises gum base in the range of 10-80% by weight of the chewing gum composition, preferably in the range 20-70% by weight, and even more preferably in the range 30-60% by weight of the chewing gum composition.

The chewing gum base, which is admixed with chewing gum ingredients as defined below, can vary substantially depending on the particular product to be prepared and on the desired masticatory and other sensory characteristics of the final product. However, typical ranges (weight %) of the above gum base components are: 5 to 50% by weight elastomeric compounds, 5 to 55% by weight elastomer plasticizers, 0 to 50% by weight filter/texturiser, 5 to 35% by weight softener and 0 to 1% by weight of miscellaneous ingredients such as antioxidants, colorants, etc.

In a preferred embodiment, the gum base may comprise an elastomer. Natural elastomers may include natural rubber such as smoked or liquid latex and guayule as well as natural gums such as jelutong, lechi caspi, massaranduba balata, sorva, perillo, rosindinha, massaranduba chocolate, chicle, nispero, gutta hang kang, and combinations thereof. Useful synthetic elastomers include, but are not limited to, synthetic elastomers listed in U.S. Food and Drug Administration, CFR, Title 21, Section 172,615, the Masticatory Substances, Synthetic, the contents of which are incorporated herein by reference for all purposes) such as polyisobutylene. e.g.having an average molecular weight in the range of about 10,000 to 1,000,000 including the range of 50,000 to 80,000, isobutylene-isoprene copolymer (butyl elastomer), styrene- butadiene copolymers e.g. having styrene-butadiene ratios of about 1:3 to 3:1, polyvinyl acetate (PVA), e.g. having a average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer and combinations hereof.

It is possible to combine a synthetic elastomer having a high molecular weight and a synthetic elastomer having a low molecular weight elastomer in a gum base. Presently preferred combinations of synthetic elastomers include, but are not limited to, polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Typically, the gum base comprises at least one elastomer in an amount in the range of 3-80% by weight of the gum base, preferably in an amount in the range of 4-60% by weight of the gum base, and even more preferred in the range of 5-40% by weight of the gum base, such as in the range of 8-20% by weight of the gum base.

Particularly interesting elastomeric or resinous polymer compounds which advantageously can be used in accordance with the present invention include polymers which, in contrast to currently used elastomers and resins, can be degraded physically, chemically or enzymatically in the environment after use of the chewing gum, thereby giving rise to less environmental pollution than chewing gums based on non-degradable polymers, as the used degradable chewing gum remnants will eventually disintegrate and/or can be removed more readily by physical or chemical means from the site where it has been dumped.

In preferred embodiments, the gum base of the chewing gum tablet may comprise one or more resins contributing to obtain the desired masticatory properties and acting as plasticizers for the elastomers of the gum base. The resin may be a natural resin and/or it may be a synthetic resin. In the present context, useful resins include, but are not limited to, natural rosin esters, often referred to as ester gums including as examples glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins and pentaerythritol esters of rosins. Other useful resinous compounds include synthetic resins such as terpene resins derived from alpha-pinene, beta-pinene, and/or d-limonene, natural terpene resins; and any suitable combinations of the foregoing. The choice of resins will vary depending on the specific application, and on the type of elastomer(s) being used.

Usually, the gum base comprises at least one resin in an amount in the range of 10-90% by weight of the gum base, preferably in the range of 20-80% by weight, even more preferred in the range of 30-70% by weight of the gum base, such as in the range of 40-60% by weight of the gum base. In preferred embodiments, the gum base comprises at least one resin in the range of 3-80% by weight of the gum base, preferably in an amount in the range of 4-60% by weight of the gum base, and even more preferred in the range of 5-40% by weight of the gum base, such as in the range of 8-20% by weight of the gum base.

The gum base may furthermore comprise one or more softener. According to the present text, the term "softener" may be interchangeably with plasticizers and plasticizing agents, and is used for ingredients, which softens the gum or chewing gum formulation and encompass wax, fat, oil, emulsifiers, surfactants, solubilizers etc. The softeners may also include sucrose polyesters, such as glycerin, lecithin, and combinations thereof. Aqueous sweetener solutions such as those containing sorbitol, hydrogenated starch hydrolysates, corn syrup and combinations thereof, may also be used as softeners and binding agents in the chewing gum according to the invention.

In a preferred embodiment, the gum base comprises an emulsifier, which aid in dispersing any immiscible components into a single stable system. The emulsifiers useful in this invention include glyceryl monostearate, lecithin, fatty acid monoglycerides, diglycerides, propylene glycol monostearate, and the like, and mixtures thereof. The emulsifier may be employed in an amount in the range of 1-15% by weight of the gum base, and preferably in the range 5-10% by weight of the gum base.

Further examples of useful emulsifier include anionic, cationic, amphoteric or nonionic emulsifiers can be used. Suitable emulsifiers include lecithins, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters ofinteresterified castor oil acid (E476), sodium stearoyliatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable emulsifiers are polyoxyethylene stearates, such as for instance polyoxyethylene (8) stearate and polyoxyethylene (40) stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllactylate, sodium laurylsulfate, polyoxyethylated hydrogenated castor oil, blockcopolymers of ethylene oxide and propyleneoxide and polyoxyethylene fatty alcohol ether. The emulsifiers may either be a single compound or a combination of several compounds.

Some emulsifier also referred to as plasticizers, to provide a variety of desirable textures and consistency properties, Because of the low molecular weight of these components, the plasticizers are able to penetrate the fundamental structure of the gum base making it plastic and less viscous. Useful plasticizers include lanolin, palmitic acid, oleic acid, stearic acid, sodium stearate, potassium stearate, glyceryl triacetate, glyceryl lecithin, glyceryl monostearate, propylene glycol monostearate, acetylated monoglyceride, glycerine, and the like, and mixtures thereof.

In preferred embodiments, the softener used in the gum base of the chewing gum of the invention is a fat. The fat may e.g. include partially or fully hydrogenated vegetable or animal fats, such as partially or fully hydrogenated coconut oil, partially or fully hydrogenated palm oil, partially or fully hydrogenated palm kernel oil, partially or fully hydrogenated rapeseed oil, partially or fully hydrogenated castor oil, partially or fully hydrogenated maize oil, partially or fully hydrogenated cottonseed oil, partially or fully hydrogenated olive oil, partially or fully hydrogenated sunflower oil, partially or fully hydrogenated safflower oil, partially or fully hydrogenated sesame oil, partially or fully hydrogenated soybean oil, partially or fully hydrogenated beef tallow, and partially or fully hydrogenated lard, and any mixture thereof and any derivative thereof. In useful embodiments, the gum base comprises a fat in an amount in the range of 1-15% by weight of the gum base, and preferably in the range 5-10% by weight of the gum base.

The gum base may furthermore comprise a wax. When a wax is present in the gum base, it softens the polymeric elastomer mixture and improves the elasticity of the gum base. The waxes employed will have a melting point below about 60°C, and preferably between about 45°C and about 55°C. The low melting wax may be a paraffin wax. The wax may be present in the gum base in an amount from about 6% to about 10%, and preferably from about 7% to about 9.5% by weight of the gum base.

In addition to the low melting point waxes, waxes having a higher melting point may be used in the gum base in amounts up to about 5%, by weight of the gum base. Such high melting waxes include beeswax, vegetable wax, candelilla wax, canauba wax, most petroleum waxes, and the like, and mixtures thereof.

Further useful waxes include natural and synthetic waxes, hydrogenated vegetable oils, petroleum waxes such as polyurethane waxes, polyethylene waxes, paraffin waxes, microcrystalline waxes, fatty waxes, sorbitan monostearate, tallow, propylene glycol, mixtures thereof, and the like, may also be incorporated into the gum base,

Anhydrous glycerin may also be employed as a softening agent, such as the commercially available United States Pharmacopeia (USP) grade. Glycerin is a syrupy liquid with a sweet warm taste and has a sweetness of about 60% of that of cane sugar, Because glycerin is hygroscopic, the anhydrous glycerin may be maintained under anhydrous conditions throughout the preparation of the chewing gum composition.

In an embodiment of the invention, the gum base comprises at least one resin in an amount in the range of 10-90% by weight of the gum base, at least one elastomer in an amount in the range of 4-60% by weight of the gum base, and an emulsifier in an amount in the range of 1-15% by weight. Preferably, the gum base comprises at least one resin in an amount in the range of 30-70% by weight of the gum base, at least one elastomer in an amount in the range of 5-40% by weight of the gum base, and an emulsifier in an amount in the range of 5-10% by weight of the gum base.

In a preferred embodiment, the gum base of the chewing gum according to the invention comprises a filler. The fillers/texturizers may include magnesium and calcium carbonate, sodium sulphate, ground limestone, silicate types such as magnesium and aluminium silicate, kaolin, clay, aluminium oxide, silicium oxide, talc, titanium oxide, mono-, di- and tri-calcium phosphates, cellulose polymers, such as wood, and combinations thereof.

The fillers/texturizers may also include natural organic fibres such as fruit vegetable fibres, grain, rice, cellulose and combinations thereof.

### Chewing gum ingredients

In accordance with the present invention the chewing gum tablet comprises one or more further chewing gum ingredients. Such a chewing gum ingredient may be selected from the group consisting least a bulk sweetener, a high intensity sweetener, a flavouring agent, a cooling agent, a warming agent, a colouring agent, a binding agent, a pH regulating agent and an active ingredient.

In a useful embodiment of the present invention, the at least one chewing gum ingredients is a bulk sweetener. The bulk sweetener may be selected from the group consisting of monosaccharides, disaccharides, polysaccharides, sugar alcohols, and mixtures thereof; randomly bonded glucose polymers such as those polymers distributed under the tradename POLYDEXTROSE by Pfizer, Inc., Groton, Conn.; isomalt (a racemic mixture of alpha-D-glucopyranosyl-1,6-mannitol and alpha-D-glucopyranosyl-1,6-sorbitol manufactured under the tradename PALATINIT by Süddeutsche Zucker), maltodextrins; hydrogenated starch hydrolysates; hydrogenated hexoses; and hydrogenated disaccharides,

Furthermore, the bulk sweetener may be selected from the group consisting of dextrose, sucrose, lactose, xylitol, mannitol, sorbitol, mannitol, maltitol, isomaltol or isomalt, erythritol, lactitol, and cyclodextrin,

In an especially preferred embodiment of the invention, the bulk sweetener is present in amount ranging from 10-70% by weight of the chewing gum composition.

The bulk sweetener may be present in amount ranging from 30-70% by weight of the chewing gum composition, such as e.g. in the range 35-65% by weight of the chewing gum composition, and in the range 40-60% by weight of the chewing gum composition. For example, the bulk sweetener may be present in amount ranging from 20-55% by weight of the chewing gum composition, such as e.g. in amount ranging from 30-50% by weight of the chewing gum composition.

In interesting embodiment, the chewing gum composition according to the invention further comprises a high intensity sweetener, Useful high intensity sweetener may be selected from the group consisting of sucralose, neotame, aspartame, salts of acesulfame, alitame, Saccharin and its salts, cyclamic acid and its salts, glycyrrhizin, dihydrochalcones e.g. NHDC, thaumatin, monellin, stevioside, Twinsweet (aspartame-acesulfame salt) and combinations thereof.

In order to provide longer lasting sweetness and flavour perception, it may be desirable to encapsulate or otherwise control the release of at least a portion of the artificial sweetener. Likewise, encapsulation may be applied for the purpose of stabilizing the ingredients. Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided e.g. using another chewing gum component, such as a resinous compound, as the encapsulation agent.

Usage level of the artificial sweetener will vary considerably depending e.g. on factors such as potency of the sweetener, rate of release, desired sweetness of the product, level and type of flavour used and cost considerations. Thus, the active level of artificial sweetener may vary from about 0.02 to 8% by weight. When carriers used for encapsulation are included, the usage level of the encapsulated sweetener will be proportionally higher. Combinations of sugar and/or non-sugar sweeteners can be used in the chewing gum formulation processed in accordance with the invention. Additionally, the softener may also provide additional sweetness such as with aqueous sugar or alditol solutions.

If a low calorie chewing gum tablet is desired, a low calorie bulking agent can be used. Examples of low calorie bulking agents include polydextrose, Raftilose, Raftilin, Inuline, fructooligosaccharides (NutraFlora®), palatinose oligosaccharided; guar gum hydrolysates (e.g. Sun Fiber®) or indigestible dextrins (e.g. Fibersol®). However, other low calorie-bulking agents can be used.

Flavouring agents may also be useful for the organoleptic properties in the chewing gum composition according to the invention. The flavouring agents which may be used include those flavouring agents known to the skilled artisan, such as natural and artificial flavouring agents. These flavouring agents may be chosen from synthetic flavour oils and flavouring aromatic and/or oils, oleoresins and extracts derived from plants, leaves, flowers, fruits, and so forth, and combinations thereof, Non-limiting representative flavour oils include spearmint oil, cinnamon oil, oil of wintergreen (methyl salicylate), peppermint oil, clove oil, bay oil, anise oil, eucalyptus oil, thyme oil, cedar leaf oil, oil of nutmeg, allspice, oil of sage, mace, oil of bitter almonds, and cassia oil. Also useful flavouring agents are artificial, natural and synthetic fruit flavours such as vanilla, and citrus oils including lemon, orange, lime, grapefruit, and fruit essences including apple, pear, peach, grape, strawberry, raspberry, cherry, plum, pineapple, apricot and so forth. These flavouring agents may be used in liquid or solid form and may be used individually or in admixture, Commonly used flavouring agents include mints such as peppermint, menthol, spearmint, artificial vanilla, cinnamon derivatives, and various fruit flavouring agents, whether employed individually or in admixture.

Other useful flavouring agents include aldehydes and esters such as cinnamyl acetate, cinnamaldehyde, citral diethylacetal, dihydrocarvyl acetate, eugenyl formate, p-methylamisol, and so forth may be used. Generally any flavoring agent or food additive such as those described in Chemicals Used in Food Processing, publication 1274, pages 63-258, by the National Academy of Sciences, may be used. This publication is incorporated herein by reference.

Further examples of aldehyde flavouring agents include, but are not limited to, acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavours), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, i.e., melonal (melon), 2,6-dimethyloctanal (green fruit), and 2-dodecenal (citrus, mandarin), cherry, grape, strawberry shortcake, and mixtures thereof.

In some embodiments, the flavouring agent may be employed in either liquid form and/or dried form. When employed in the latter form, suitable drying means such as spray drying the oil may be used. Alternatively, the flavouring agent may be absorbed onto water soluble materials, such as cellulose, starch, sugar, maltodextrin, gum arabic and so forth or may be encapsulated. The actual techniques for preparing such dried forms are well-known.

In some embodiments, the flavouring agents may be used in many distinct physical forms well-known in the art to provide an initial burst of flavour and/or a prolonged sensation of flavour. Without being limited thereto, such physical forms include free forms, such as spray dried, powdered, beaded forms, encapsulated forms, and mixtures thereof.
The amount of flavouring agent employed herein may be a matter of preference subject to such factors as the type of final chewing gum, the individual flavour, the gum base employed, and the strength of flavour desired. Thus, the amount of flavouring may be varied in order to obtain the result desired in the final product and such variations are within the capabilities of those skilled in the art without the need for undue experimentation. In chewing gum compositions, the flavouring agent is generally present in amounts from about 0.02% to about 5% by weight, and more specifically from about 0.1% to about 2% by weight, and even more specifically, from about 0.8% to about 1.8%, by weight of the chewing gum composition.

According to the invention, encapsulated flavours may be added to the final blend prior to compression. Different methods of encapsulating flavours mixed into the gum base and flavours compressed into the chewing gum may e.g. include spray drying, spray cooling, film coating, coascervation, double emulsion method (extrusion technology) or prilling. Materials to be used for the above-mentioned encapsulation methods may e.g. include gelatine, wheat protein, soya protein, sodium caseinate, caseine, gum arabic, modified starch, hydrolyzed starches (maltodextrines), alginates, pectin, carregeenan, xanthan gum, locus bean gum, chitosan, bees wax, candelilla wax, camauba wax, hydrogenated vegetable oils, zein and/or sucrose.

Useful cooling agents are mentioned in U.S. Patent No. 6,627,233, the contents of which are incorporated herein by reference for all purposes. Particular examples of cooling agents include: menthol, xylitol, menthane, menthone, menthyl acetate, menthyl salicylate, N,2,3-trimethyl-2-isopropyl butanamide (WS-23), substituted p-menthanes, substituted p-menthane-carboxamides (e.g., N-ethyl-p-menthane-3-carboxamide (FEMA 3455)), acyclic carboxamides, substituted cyclohexanamides, substituted cyclohexane carboxamides, substituted ureas and sulphonamides, and substituted menthanols (all from Wilkinson Sword); hydroxymethyl and hydroxyethyl derivatives of p-menthane (from Lever Bros.); menthyl succinate; 2-mercapto-cyclo-decanone (from International Flavors and Fragrances); 2-isopropanyl- 5-methylcyclohexanol (from Hisamitsu Pharmaceuticals, hereinafter "isopregol"); hydroxycarboxylic acids with 2-6 carbon atoms; menthone glycerol ketals (FEMA 3807, tradename FRESCOLAT(TM) type MGA); 3-l-menthoxypropane-1,2-diol (from Takasago, FEMA 3784, (hereinafter "TCA")); menthyl lactate; (from Haarman & Reimer, FEMA 3748, tradename FRESCOLAT(TM) type ML). These and other suitable cooling agents are further described in the following U.S. patents, all of which are incorporated in their entirety by reference hereto: U.S. Pat. Nos. 4,230,688 and 4,032,661 to Rowsell et al.; 4,459,425 to Amano et al.; 4,136,163 to Watson et al.; and 5,266,592 to Grub et al. The cooling agents are typically present in amounts of about 0.001 to about 10% by weight of the chewing gum composition.

Useful warming agents may be selected from a wide variety of compounds known to provide the sensory signal of warming to the user. These compounds offer the perceived sensation of warmth, particularly in the oral cavity, and often enhance the perception of flavours, sweeteners and other organoleptic components. Among the useful warming compounds included are vanillyl alcohol n-butylether (TK-1000) supplied by Takasago Perfumary Company Limited, Tokyo, Japan, vanillyl alcohol n-propylether, vanillyl alcohol isopropylether, vanillyl alcohol isobutylether, vanillyl alcohol n-aminoether, vanillyl alcohol isoamyleather, vanillyl alcohol n-hexyleather, vanillyl alcohol methylether, vanillyl alcohol ethyleather, gingerol, shogaol, paradol, zingerone, capsaicin, dihydrocapsaicin, nordihydrocapsaicin, homocapsaicin, homodihydrocapsaicin, ethanol, isopropol alcohol, iso-amylacohol, benzyl alcohol, glycerine, and combinations thereof. Furthermore, useful warming agents include capsicum and nicotinate esters, such as benzyl nicotinate.

Whiteners and colouring agents may be used in amounts effective to produce the desired colour. The colouring agents may include pigments which may be incorporated in amounts up to about 6%, by weight of the chewing gum composition. For example, titanium dioxide may be incorporated in amounts up to about 2%, and preferably less than about 1%, by weight of the chewing gum composition. The colourants may also include natural food colours and dyes suitable for food, drug and cosmetic applications. These colourants are known as F.D.& C. dyes and lakes. The materials acceptable for the foregoing uses are preferably water-soluble. Illustrative nonlimiting examples include the indigoid dye known as F.D,& C. Blue No. 2, which is the disodium salt of 5,5-indigotindisuffonic acid. Similarly, the dye known as F.D.& C. Green No. 1 comprises a triphenylmethane dye and is the monosodium salt of 4-[4-(N-ethyl-p-sulfoniumbenzylamino) diphenylmethylene]-[1-(N-ethyl-N-p- sulfoniumbenzyl)-delta-2,5-cyclohexadieneimine]. A full recitation of all F.D.&C. colourants and their corresponding chemical structures may be found in the Kirk-Othmer Encyclopedia of Chemical Technology, 3rd Edition, in volume 5 at pages 857-884, which text is incorporated herein by reference.

Useful pH-regulating agents, acidity regulators, or pH control agents are additives which may be added to the chewing gum to change or maintain pH (acidic, alkaline or pH neutral). They can be organic or mineral acids (acidulants), bases, neutralizing agents, or buffering agents. Examples of useful compounds include ascorbic acid, fumaric acid, adipic acid, lactic acid, malic acid, citric acid, tartaric acid, propionic acid, phosphoric acid and combinations thereof.

Compression adjuvants may also be added. These compounds facilitate compression of the gum into tablets. Suitable compression adjuvants include, but are limited to, glidants, lubricants, wetting agents, diluents, humectants. More specifically, useful compression adjuvants include silicon dioxide, magnesium stearate, calcium stearate, behenic acid, talc and similar substances which can be used to limit the tendency of the gum tablets to stick to the presses.

The above mentioned chewing gum ingredients may be pre-mixed into the gum base or be added to a portion of the chewing gum comprising no or a low amount of gum base.

In an embodiment of the invention, the chewing gum comprises a center filling. Furthermore, the chewing gum tablet may be processed into in a number of different shapes such as a stick, a core, a tablet, a slab, a bead, a pellet, a tape, or a ball.

### Alkalizing agent

Preliminary experiments (not shown here) have indicated that compressed chewing gum tablets according to the present invention gain improved stability by the presence of one or more alkalizing agent. Thus, in a preferred embodiment of the invention, the compressed chewing gum tablet furthermore comprises one or more alkalizing agent(s).

In the context of the present invention, the term "alkalizing agent" covers any compounds which are able to increase the pH of deionized water when added to it.

In an embodiment of the invention, at least one of the one or more alkalizing agent(s) comprises an alkali or alkaline-earth metal hydroxide.

In an embodiment of the invention, at least one of the one or more alkalizing agent(s) comprises a carbonate salt.

In an embodiment of the invention, at least one of the one or more alkalizing agent(s) comprises a bicarbonate salt.

In an embodiment of the invention, at least one of the one or more alkalizing agent(s) comprises a phosphate salt.

In an embodiment of the invention, at least one of the one or more alkalizing agent(s) comprises a borate salt.

In an embodiment of the invention, at least one of the one or more alkalizing agent(s) comprises a basic salt of an organic acid.

In an embodiment of the invention, at least one of the one or more alkalizing agent(s) comprises a basic salt of a carboxylic acid or of a hydroxy carboxylic acid. An example of this is e.g. a basic salt of a food acid. Examples of useful alkalizing agents are a basic salt of ascorbic acid, a basic salt of fumaric acid, a basic salt of adipic acid, a basic salt of lactic acid, a basic salt of malic acid, a basic salt of citric acid, a basic salt of tartaric acid, a basic salt of propionic acid, or combinations thereof.

In an embodiment of the invention, at least one of the one or more alkalizing agent(s) comprises tri-sodium citrate.

The one or more alkalizing agent(s) may be located different places in the tablet. In an embodiment of the invention, the first compressed module comprises the one or more alkalizing agent(s).

In a preferred embodiment of the invention, the second compressed module comprises the one or more alkalizing agent(s).

In yet an embodiment of the invention, the first and second compressed modules comprise the one or more alkalizing agent(s), i.e, the one or more alkalizing agent may be present both in first and the second compressed module at the same time.

The stability effect provided by the alkalizing agent appears to be particularly predominant when the one or more alkalizing agent(s) is/are present in the tablet an amount sufficient to yield a pH within a specific range as mentioned below when the tablet is submerged and partially dissolved in water.

Thus, in a preferred embodiment of the invention, the one or more alkalizing agent(s) is/are present in the tablet an amount sufficient to form a pH in the range of pH 5-12, preferably in the range of pH 5.5-11, and even more preferably in the range of pH 6-10, such as in the range of pH 6.5-9, or in the range of pH7-9,
wherein the formed pH is determined by submerging the compressed chewing gum tablet chewing gum tablet in 50 mL deionized water, stirring the mixture of the tablet and deionized water for 30 minutes, and then immediately measuring the pH of the mixture, and wherein the temperature of the mixture is maintained at approx. 25 degrees C during the stirring and the measurement.

In embodiments where the tablet comprises a water-insoluble coating, the pH determination is performed using the uncoated tablet.

In another embodiment of the invention, the one or more alkalizing agent(s) is/are present in the second compressed module in an amount sufficient to form a pH in the range of pH 5-12, preferably in the range of pH 5.5-11, and even more preferably in the range of pH 6-10, such as in the range of pH 6.5-9, or in the range of pH 7-9,
wherein the formed pH is determined by submerging the second compressed module in 50 mL deionized water, stirring the mixture of the second compressed module and deionized water for 30 minutes, and then immediately measuring the pH of the mixture, and wherein the temperature of the mixture is maintained at approx. 25 degrees C during the stirring and the measurement.

The amount of the one or more alkalizing agent(s) varies with the selection of the active compound according to formula I and the other excipients of the tablet. In an embodiment of the invention, the compressed chewing gum tablet comprises the one or more alkalizing agent(s) in an amount in the range of 0.01-25% by weight of the tablet, preferably in the range of 0.1-10% by weight of the tablet, and even more preferred in the range of 0.25-5% by weight of the tablet.

In yet an embodiment of the invention, the first compressed layer comprises the one or more alkalizing agent(s) in an amount in the range of 0.01-25% by weight of the first compressed layer, preferably in the range of 0.1-10% by weight of the first compressed layer, and even more preferred in the range of 0.25-5% by weight of the first compressed layer,

In preferred embodiment of the invention, the second compressed layer comprises the one or more alkalizing agent(s) in an amount in the range of 0.01-25% by weight of the second compressed layer, preferably in the range of 0.1-10% by weight of the second compressed layer, and even more preferred in the range of 0.25-5% by weight of the second compressed layer.

While the one or more alkalizing agent(s) may be present in the tablet in many different forms, it is presently preferred that at least one of the one or more alkalizing agents(s) is in particulate form.

In another embodiment of the invention, at least one of the one or more alkalizing agents(s) is in intimate contact with the active compound according to formula I. Intimate contact may e.g. be accomplished by granulated the at least one of the one or more alkalizing agents(s) with the active compound according to formula I. Alternatively it may be accomplished by pre-blending the at least one of the one or more alkalizing agents(s) with the active compound according to formula I with the active compound according to formula I before the preparing the powder mixture for compression. Pre-blending is particularly effective if the average particle size of the alkalizing agent is substantially smaller than the particle size of the particles comprising the active compound according to formula I.

### Tablet material

In accordance with the present invention, the second and/or third compressed module of the chewing gum may comprise tablet material. The expression "tablet material" is in the present context used for the above described chewing gum ingredients when these are used in a compressed module comprising tablet material. However, examples of further useful tablet materials include, but are not limited to, conventional pharmaceutical acceptable excipients such as a glidant, a lubricant, a filler substance, and a dry or wet binder.

Examples of useful glidants and lubricants are stearic acid, metallic stearates, talc, colloidal silica, sodium stearyl fumarate and alkyl sulphates.

In the present invention, a dry binder such as e.g. sorbitol, isomalt, or mixture thereof may be used. The dry binder provides the effect of binding a material and thereby providing a powder that can be compressed into a tablet.

A wet binder is an excipient that in combination with water facilitates a powder to be compressed into tablets. A wet binder must, at least to some extent, be soluble in water. Examples of wet binders are PVP (polyvinylpyrrolidone), HPMC (hydroxymethylpropylcellulose) or gelatine.

A filler substance may be any pharmaceutically acceptable substance that does not interact with the active compound according to formula I or with other excipients. Useful filler substances include sorbitol, mannitol, dextrins, maltodextrins, inositol, erythritol, isomalt, lactitol, maltitol, mannitol, xylitol, low-substituted hydroxypropylcellulose, starches or modified starches (e.g. potato starch, maize starch, rice starch, pre-gelatinised starch), polyvinylpyrrolidone, polyvinylpyrrolidone/vinyl acetate copolymer, agar (e.g. sodium alginate), carboxyalkylcellulose, dextrates, gelatine, gummi arabicum, hydroxypropyl hydroxypropytmethylcellulose, methylcellulose, microcrystalline cellulose, polyethylene glycol, polyethylene oxide, polysaccharides e.g. dextran, soy polysaccharide, sodium carbonate, and sodium chloride.

### Coating

In accordance with the invention, the chewing gum tablet may comprise a coating applied onto the chewing gum center. In the present context, a suitable coating is any coating that results in extended storage stability of the compressed chewing gum products as defined above, relative to a chewing gum of the same composition that is not coated. Thus, suitable coating types include hard coatings, soft coatings, film coatings and sealing coatings of any composition including those currently used in coating of chewing gum, pharmaceutical products and confectioneries. The chewing gum tablet comprises the coating in an amount in the range of 1-80% by weight of the chewing gum, such as in an amount in the range of 10-50%, or 15-45% by weight of the chewing gum. Preferably, the chewing gum tablet comprises the coating in an amount in the range of 20-40% by weight of the chewing gum tablet.

In a useful embodiment of the invention, the coating comprises an active compound according to formula I. The coating may e.g. comprise an active compound according to formula I in an amount in the range of 1-30 mg, and preferably in the range of 5-20 mg. Preferably, the coating comprises an active compound according to formula I an amount in the range of 1-10% by weight of the coating.

The coating may be a hard coating, which term is used in the conventional meaning of that term including sugar coatings and sugar-free (or sugarless) coatings and combinations thereof. The objects of hard coating are to obtain a sweet, crunchy layer, which is appreciated by the consumer, and to protect the composition for various reasons. In a typical process of providing the composition with a protective sugar coating the gum centers are successively treated in suitable coating equipment with aqueous solutions of crystallizable sugar such as sucrose or dextrose, which, depending on the stage of coating reached, may contain other functional ingredients, e.g. fillers, colours, etc. In the present context, the sugar coating may contain further functional or active compounds including flavour compounds, pharmaceutically active compounds and/or polymer degrading substances.

In the production of chewing gums it may, however, be preferred to replace the cariogenic sugar compounds in the coating by other, preferably crystallizable, sweetening compounds that do not have a cariogenic effect. In the art such coating is generally referred to as sugarless or sugar-free coatings. Presently preferred non-cariogenic hard coating substances include polyols, e.g. sorbitol, maltitol, mannitol, xylitol, erythritol, lactitol, isomalt and tagatose which are obtained by industrial methods by hydrogenation of D-glucose, maltose, fructose or levulose, xylose, erythrose, lactose, isomaltulose and D-galactose, respectively. One advantage of using polyols in the coating is that they act simultaneously as a sweetener and as a taste-masking agent for the bitter taste of an active compound according to formula I.

In a typical hard coating process, a syrup containing crystallizable sugar and/or polyol is applied onto the chewing gum tablet and the water it contains is evaporated off by blowing with warm, dry air. This cycle may be repeated several times, typically 10 to 80 times, in order to reach the swelling required. The term "swelling" refers to the increase in weight of the products, as considered at the end of the coating operation by comparison with the beginning, and in relation to the final weight of the chewing gum.

Alternatively, the coating may be a soft coating. Such a soft coating is applied using conventional methods and may advantageously consist of a composition of a sugar or any of the above non-cariogenic, sugar-less sweetening compounds and a starch hydrolysate.

In a preferred embodiment of the invention, the chewing gum tablet comprise a film coating. The film coating may be obtained by subjecting the composition to a film coating process and which therefore comprises one or more film-forming polymeric agents and optionally one or more auxiliary compounds, e.g. plasticizers, pigments and opacifiers. A film coating is a thin polymer-based coating applied to a composition of any of the above forms. The thickness of such a film coating is usually between 20 and 100 micrometer. Generally, the film coating is obtained by passing the composition through a spray zone with atomized droplets of the coating materials in a suitable aqueous or organic solvent vehicle, after which the material adhering to the composition is dried before the next module of coating is received. This cycle is repeated until the coating is complete.

In the present context, suitable film-coating polymers include edible cellulose derivatives such as cellulose ethers including methylcellulose (MC), hydroxyethyl cellulose (HEC), hydroxypropyl cellulose (HPC) and hydroxypropyl methylcellulose (HPMC). Other useful film-coating agents are acrylic polymers and copolymers, e.g. methylacrylate aminoester copolymer or mixtures of cellulose derivatives and acrylic polymers. Useful polymers may include: cellulose acetate phtalate (CAP), polyvinyl acetate phtalate (PVAP), shellac, metacrylic acid copolymers, cellulose acetate trimellitate (CAT) and HPMC. It will be appreciated that the outer film coating according to the present invention may comprise any combination of the above film-coating polymers.

In other embodiments of the invention, the film-coating layer of the chewing gum tablet comprise a plasticizing agent having the capacity to alter the physical properties of a polymer to render it more useful in performing its function as a film forming material. In general, the effect of plasticizers will be to make the polymer softer and more pliable as the plasticizer molecules interpose themselves between the individual polymer strands thus breaking down polymer-polymer interactions. Most plasticizers used in film coating are either amorphous or have very little crystallinity.

In the present context, suitable plasticizers include polyols such as glycerol, propylene glycol, polyethylene glycol, e.g. the 200-6000 grades hereof, organic esters such as phtalate esters, dibutyl sebacate, citrate esters and triacetin, oils/glycerides including castor oil, acetylated monoglycerides and fractionated coconut oil.

The choice of film-forming polymer (s) and plasticizing agent (s) for the film coating of the composition is made with due consideration for achieving the best possible barrier properties of the coating in respect of dissolution and diffusion across the film of moisture and gasses.

The film coating of the chewing gum tablet may also contain one or more colorants or opacifiers. In addition to providing a desired colour hue, such agents may contribute to protecting the compressed gum base against pre-chewing reactions, in particular by forming a barrier against moisture and gasses. Suitable colorants/opacifiers include organic dyes and their lakes, inorganic colouring agents, e.g. titanium oxide and natural colours such as e.g. beta-carotene.

Additionally, film coatings may contain one or several auxiliary substances such as flavours and waxes or saccharide compounds such as polydextrose, dextrins including maltodextrin, lactose, modified starch, a protein such as gelatine or zein, a vegetable gum and any combination thereof.

A sealing coating of e.g. shellac, ethyl cellulose, zein, acrylic compounds or carnauba wax or the like may be applied over the hard coating, if desired, in order to seal the crunchy coating to reduce the exposure of the coating to atmospheric moisture.

The coating, in general, typically comprises one or more layers. For example the number of layers of the coating may be in the range of 1-100 layers, such as 3-75 layers, 10-60 layers, and 20-40 layers.

The coating comprises for example comprise a wax layer. In an embodiment of the invention, the outermost layer of the coating is a wax layer.

A compressed chewing gum tablet according to the present invention, has typically a weight in the range of 0.1-10 g, such as in the range of 0.5-5 g or in the range of 0.75-2.5 g, preferably in the range of 0.8-2 g, and even more preferred in the range of 1-1.5 g. Furthermore, the chewing gum tablet has a weight in the range of 0.5-3,0 g, such as in the range of 0.75-2.5 g or in the range of 0.8-2.0 g, preferably in the range of 1.0-1.5 g. Center-filled chewing gums normally have weights in the range of 0.5-5 g, preferably in the range of 1-4 g, and even more preferred in the range of 2-3 g. Typical weights for bead shaped chewing gums are in the range of 0.1-0.6 g, preferably in the range of 0.2-0.5 g, and even more preferred in the range of 0.3-0.4 g.

Yet an aspect of the invention relates to an oral dosage form identical to the compressed chewing gum tablet as defined herein, but with the one exception that all gum base has been replaced with an inert excipient, such as Polyol sweetener. All aspects and embodiments mentioned herein, except for details relating to the gum base, also apply to the oral dosage form.

The oral dosage form may be in the form of a capsule; a tablet, and particularly an effervescent tablet or a fast disintegrating tablet; a liquid syrup; a dry syrup; a lozenge; or a hardboiled confectionery. In a preferred embodiment of the invention, the oral dosage form is in the form of a tablet.

It should be understood that any embodiments and/or feature discussed above in connection with the compressed chewing gum tablet according to the invention apply by analogy to the below aspects of the present invention.

### Method of preparing a compressed chewing gum tablet

In further aspects there are provided methods for preparing a compressed chewing gum tablet having multiple compressed modules. Initially, chewing gum particles containing gum base are provided. Useful particles may be manufactures according to conventional methods or e.g. those described in the EP 1 474 993, EP 1 474 994 and EP 1 474 995, hereby incorporated by reference.

The chewing gum particles may be in any suitable form according to the invention. As described above, in some embodiments, the particles have been particulated prior to application. Particulation may be in any form of "building up" particles from smaller primary particles into macro particles or in any form of "building down" from larger substances into macro particles. Any form of particulation may be applied, such as granulation, pelletizing, agglomeration, or any other suitable means for particulation.

Granulation may be applied in some embodiments as a means for particulation, resulting in granules. Granules should be understood in its broadest content. In some embodiments of the invention, the granules may be a result of a total chewing gum manufacture, where the chewing gum after production is comminuted into smaller particles, optionally under cooling conditions such as with a coolant or physical cooling, where after these particles are pressed together, optionally using at least some further processing aids. The comminuted particles may be achieved by grinding, milling, or any other suitable processing means.

Thus, in a specific embodiment the chewing gum particles are provided by a method where the particles are obtained through grinding of the prepared chewing gum composition. More specifically, such a method comprises the steps of a) mixing of a soft basic gum base with at least one sweetener and, optionally, at least one other chewing gum ingredient, at a temperature of between 35 and 75°C; b) cooling of the mixture thus obtained to a temperature of between 0 and -40°C and, preferably, between -10 and -40°C; c) grinding and subsequent screening of the mixture thus obtained to a particle size of less than 10 mesh; and d) optional mixing of the powder thus obtained with at least one anti-agglutination agent.

Agglomeration may also be applied in some other embodiments as a means for participation, resulting in agglomerates.

Pelletizing may be applied in some other embodiments as a means for particulation, resulting in pellets. The pellets may be partly manufactured as a result of an extruding process. In some embodiments, the pellets are pelletized in an underwater process, whereby gum base are pressed through dies in a die plate, meaning openings of a certain diameter, into a cooling media and thereupon dried. In some other embodiments, the pellets are pelletized in a strand pelletizing process with cool air.

Thus, in a specific embodiment, the chewing gum particles containing gum base are provided by a method comprising at least the steps of a) feeding a gum base into an extruder; b) pressurizing the gum base in the extruder; c) extruding the gum base through a die means; and d) cutting the extruded gum base in a liquid filled chamber.

In useful embodiments, the provided chewing gum particles are made entirely of a
gum base, substantially without conventional chewing gum ingredients. In this case, the chewing gum ingredients may be applied in the compression process, such as by adding the chewing gum ingredients together with the gum base particles for compression.

However, under some circumstances it may be useful to provide chewing gum particles made entirely of a chewing gum composition, substantially without further needs for chewing gum ingredients in the compression process.

Chewing gum ingredients, e.g. flavours and sweeteners, may with advantage be added to the gum base in order to obtain a gum base composition in the extruder immediately before the composition is extruded through the die means into the water filled chamber where the extruded and cut chewing gum composition is immediately cooled to low temperatures.

Of course, intermediate solutions may be applicable, such as a varying amount of chewing gum ingredients in the chewing gum particles or in the compression process. It may be preferred to apply at least a certain amount of high intensity sweetener and/or flavour and/or colour to the chewing gum particles in some embodiments of the invention, such as in case the chewing gum particles substantially consist of gum base.

By adding the chewing gum ingredients to the chewing gum particles, the ingredients are only subjected to elevated temperatures during the extrusion, such as only during the latter part thereof, and the short duration of the extrusion and the quick cooling in the water prevents or reduces decomposition of fragile flavours components, and thus preserving a maximum of the components. This is especially important for natural flavours in order to maintain the full natural taste of the flavour.

In accordance with the present invention, the chewing gum tablet is a compressed chewing gum tablet. The compression is preferably performed by applying pressure to the mixture of chewing gum particles, ingredients etc., whereby the bulk volume is reduced and the amount of air is decreased. During this process energy is consumed. As the components of the mixture come into closer proximity to each other during the volume reduction process, bonds may be established between the components. The formation of bonds is associated with a reduction in the energy of the system as energy is released. Volume reduction takes place by various mechanisms and different types of bonds may be established between the components depending on the pressure applied and the properties of the components.

In one aspect of the present invention, there is provided a method of preparing a compressed chewing gum tablet, comprising one compressed module, the method comprising the steps of: a) providing a portion comprising an active compound according to formula I, a portion comprising taste-masking agent, and chewing gum containing gum base; b) optionally providing one or more further chewing gum ingredients; c) dosing the portion comprising the compound according to formula I, the portion comprising taste-masking agent, and the chewing gum particles containing gum base, and optionally the one or more further chewing gum ingredients; and d) compressing a) an b) after dosing, to obtain a first compressed module.

Thus, the compressed chewing gum tablet is prepared by providing a portion comprising the compound according to formula I, a portion comprising taste-masking agent, and chewing gum particles containing gum base. Subsequent, the portions are individually dosed, i.e. the portions are individually loaded in the table machine, and compressed together under high pressure (typically when applying cooling) into a first compressed module. Any tablet pressing machine may be used which is capable of pressing tablets comprising particles containing chewing gum base,

In accordance with the present invention, one or more chewing gum ingredients may, as described above, may be provided and compressed together in step d) with the portion comprising the compound according to formula I, the portion comprising taste-masking agent and the chewing gum particles containing gum base. However, the one and more chewing gum ingredients may also be added to the gum base in the extruder as described above.

In a further aspect of the present invention, the method comprising the steps of a) providing a portion comprising an active compound according to formula I, a portion comprising taste-masking agent, and chewing gum particles containing gum base; b) optionally providing one or more further chewing gum ingredients; c) mixing the portion comprising the compound according to formula I, the portion comprising taste-masking agent, and the chewing gum particles containing gum base, and optionally the one or more further chewing gum ingredients, thus obtaining a mixture, and d) compressing the mixture, to obtain a first compressed module. Thus, the portions of the chewing gum components are mixed before the loading of the tablet machine.

In a useful embodiment, the methods according to the invention furthermore comprise a step of coating the first compressed module with the above mentioned coatings.

In an embodiment, the above methods furthermore comprises the steps of e) providing a portion comprising tablet material; f) contacting the first compressed module with the portion of step e), i.e. the tablet material; and g) compressing the portion of e) and the first compressed module to obtain a coherent compressed chewing gum tablet comprising a first and a second compressed module. A further step of the present methods comprises a step of coating the coherent compressed chewing gum tablet of step g).

Useful tablet materials are mentioned above. Furthermore, the method comprises a step of coating the coherent compressed chewing gum tablet.

A further aspect relates to a method of preparing a compressed chewing gum tablet according to the invention comprising two compressed modules, the method comprising the steps of a) providing chewing gum particles containing gum base and optionally portion(s) comprising one or more chewing gum ingredients; b) providing a portion comprising an active compound according to formula I and a portion comprising a taste-masking agent; c) compressing a) to obtain a first compressed module; d) contacting the first compressed module with b); and e) compressing b) and the first compressed module to obtain a coherent compressed chewing gum tablet comprising a first compressed module and a second compressed module. It will be understood, that the portion comprising an active compound according to formula I and the portion comprising a taste-masking agent may be dosed individually or mixed together before dosed in the tablet machine.

A further step of the present method comprises a step of coating the coherent compressed chewing gum tablet of step e).

In a useful embodiment, chewing gum particles containing gum base and optionally one or more chewing gum ingredients are further provided in step b), and subsequent compressed to obtain a second compressed module prior to contacting the first portion.

In an interesting embodiment, a tablet material is further provided in step b).

In a further aspect, is provided a method of preparing a compressed chewing gum tablet according to the invention comprising two compressed modules, the method comprising the steps of a) providing chewing gum particles containing gum base and a portion comprising an active compound according to formula I, and optionally portion(s) comprising one or more chewing gum ingredients; b) providing a portion comprising taste-masking agent; c) compressing a) to obtain a first compressed module; d) contacting the first compressed module with b); e) compressing b) and the first compressed module, to obtain a coherent compressed chewing gum tablet comprising a first compressed module and a second compressed module. It will be understood, that the portion comprising an active compound according to formula I and chewing gum particles comprising gum base may be dosed individually or mixed together before in the tablet machine.

A further step of the present method according comprises a step of coating the coherent compressed chewing gum tablet of step e).

In a useful embodiment, chewing gum particles containing gum base and optionally one or more chewing gum ingredients are further provided in step b), and subsequent compressed to obtain a second compressed module prior to contacting the first portion,

In an interesting embodiment, a tablet material is further provided in step b).

In a further aspect of the present invention, there is provided a method of preparing a compressed chewing gum tablet according to the invention comprising three compressed modules, the method comprising the steps of a) providing chewing gum particles containing gum base, a portion comprising a taste-masking agent, and optionally portion(s) comprising one or more chewing gum ingredients; b) providing a portion comprising tablet material and optionally a portion comprising an active compound according to formula I; c) providing a portion comprising tablet material and a portion comprising an active compound according to formula I; d) locating b) and c) on opposite sites of a) following a sequence of one or more compressing step(s), to obtain a coherent compressed chewing gum comprising a first compressed module and a second module and a third compressed module. It will be understood, that the portion comprising a taste-masking agent and the chewing gum particles containing gum base may be dosed individually or mixed together before dosed in the tablet machine.

In a useful embodiment, the method according to the invention furthermore comprises a step of coating the coherent compressed chewing gum tablet of step d).

A still further aspect relates to a method of preparing a compressed chewing gum tablet according to the invention comprising three compressed modules, the method comprising the steps of a) providing chewing gum particles containing gum base, a portion comprising an active compound according to formula I, and optionally portion(s) comprising one or more chewing gum ingredients, b) providing a portion comprising tablet material and optionally a portion comprising a taste-masking agent, c) providing a portion comprising tablet material and a portion comprising a taste-masking agent, d) locating b) and c) on opposite sites of a) following a sequence of one or more compressing step(s), to obtain a coherent compressed chewing gum tablet comprising a first compressed module and a second compressed module and a third compressed module.

In a useful embodiment, the method according to the invention furthermore comprises a step of coating the coherent compressed chewing gum tablet of step d).

A final aspect relates to a method of preparing a compressed chewing gum tablet according the invention comprising three compressed modules, the method comprising the of a) providing chewing gum particles containing gum base, and optionally portion(s) comprising one or more chewing gum ingredients; b) providing a portion comprising tablet material and a portion comprising an active compound according to formula I and a portion comprising a taste-masking agent; c) providing a portion comprising tablet material a portion comprising an active compound according to formula I and a portion comprising a taste-masking agent; and d) locating b) and c) on opposite sites of a) following a sequence of one or more compressing step(s), to obtain a coherent compressed chewing gum tablet comprising a first compressed module and a second compressed module and a third compressed module. It will be understood, that the a portion comprising an active compound according to formula I and the portion comprising a taste-masking agent may be dosed individually or mixed together before dosed in the tablet machine.

The following examples are included to demonstrate particular embodiments of the invention. However, those of skill in the art should, in view of the present disclosure, appreciate that many changes can be made in the specific embodiments which are disclosed and still obtain a like or similar result without departing from the spirit and scope of the invention. The following examples are offered by way of illustration and are not intended to limit the invention in any way.

### EXAMPLES

### EXAMPLE 1

### Compressed chewing gum tablet having one compressed module

24 specific chewing gum tablets comprising cetirizine, a polyol (taste-masking agent) and chewing gum particles containing gum base are prepared. Table 1.1 shows the location of the cetirizine and the taste-masking agent in the different chewing gum type A-F, and Table 1.2 show the concentration of cetirizine (i.e. 5mg and 10mg) and taste-masking agent (i.e. 250mg and 500 mg) in the chewing gum type A-F.

The chewing gum in the following example is manufactured from a commercially available gum base (Danfree, available from Gumlink A/S, Vejle, Denmark) supplemented with about 15% by weight elastomer, about 20% by weight natural resin, about 20% by weight PVA, about 20% by weight filler, about 5% emulsifier, and about 20% by weight fat. Such mixture is in the following referred to as the "gum base"

### Cetirizine and/or polyol between the chewing gum particles containing gum base

The gum base is feed to an extruder (Leistrits ZSE/BL 360 kw 104, available from GALA GmbH, Germany) and flavour is added and mixed with gum base in the extruder. The resulting gum base composition is extruded to a granutator comprising a die plate and liquid filled chamber (A5 PAC 6, available from GALA GmbH, Germany) connected to a water system comprising water supply for the granulator and centrifugal dryer (TWS 20, available from GALA GmbH, Germany). The granulator produces chewing gum particles containing gum base. The preparation of chewing gum particles is described in details in EP1474993, EP147994 and EP147995.

The chewing gum particles containing gum base are subsequently mixed with cetirizine, taste-masking agent and/or other chewing gum ingredients in order to obtain chewing gum tablet type D, E and F.

### Cetirizine and/or polyol in the chewing gum particles containing gum base

Method I: The gum base is feed to an extruder (Leistrits ZSE/BL 360 kw 104, available from GALA GmbH, Germany) and cetirizine, polyol and/or flavour are added and mixed to the gum base in the extruder. The resulting chewing gum composition is extruded to a granulator comprising a die plate and liquid filled chamber (A5 PAC 6, available from GALA GmbH, Germany)

Method II: Cetirizine and/or polyol may also be incorporated into the particles by adding them during the manufacturing of the gum base. Cetirizine and/or polyol are added during the mixing of the gum base ingredients preferably at the end of mixing. The gum base can subsequent be particulated by extrusion, pettetizing, milling, grinding or any other methods

### Compression

Before pressing, the mixture is passed through a standard horizontal vibration sieve removing particles larger than 2.6 mm. The mixture is subsequently passed to a standard tablet pressing machine comprising dosing apparatus (e.g. P 3200 C, available from Fette GmbH, Germany) and pressed into compressed chewing gum tablets having one compressed module, The filling depth is approximately 7.5 mm and the diameter 7.0 mm. The tablets are pre-compressed to 5.0 mm and then main compressed to 3.2 m using a pressing pressure of 33.0 - 33.6 kN. There are 61 punches on the rotor, and the rotor speed used is 11 rpm. The individual compressed tablets have a weight of approx. 1.5 g.

**Table 1.1: Location of cetirizine and polyol in compressed chewing gum tablet types A-F**

| **Chewing gum** | **Cetirizine location** | **Polyol location** |
|---|---|---|
| A | In the particles | In the particles |
| B | In the particles | Between the particles |
| C | In the particles | Both in the particles and between the particle |
| D | Between the particles | In the particles |
| | E Between the particles | Between the particles |
| F | Between the particles | Both in the particles and between the particles |

**Table 1.2: Concentration of cetirizine and polyol in compressed chewing gums types A-F**

| **No** | **Chewing** | **Conc. Cetirizine** | **Conc. Polyol** |
|---|---|---|---|
| 1 | A | 5 | 250 |
| 2 | A | 5 | 500 |
| 3 | A | 10 | 250 |
| 4 | A | 10 | 500 |
| 5 | B | 5 | 250 |
| 6 | B | 5 | 500 |
| 7 | B | 10 | 250 |
| 8 | B | 10 | 500 |
| 9 | C | 5 | 250 |
| 10 | C | 5 | 500 |
| 11 | C | 10 | 250 |
| 12 | C | 10 | 500 |
| 13 | D | 5 | 250 |
| 14 | D | 5 | 500 |
| 15 | D | 10 | 250 |
| 16 | D | 10 | 500 |
| 17 | E | 5 | 250 |
| 18 | E | 5 | 500 |
| 19 | E | 10 | 250 |
| 20 | E | 10 | 500 |
| 21 | F | 5 | 250 |
| 22 | F | 5 | 500 |
| 23 | F | 10 | 250 |
| 24 | F | 10 | 500 |

### Example 2

### Compressed chewing gum tablet having two compressed modules

A number of compressed chewing gum tablets having two compressed modules comprising cetirizine and polyol (taste-masking agent) are prepared. Tables 2.1, 2.3, 2.5 and 2.7 show the location of the cetirizine and polyol in the different chewing gum type G-R, S-DD, EE-NN and OO-WW, respectively, and Tables 2.2, 2.4, 2.6 and 2.8 show the concentration of cetirizine (i.e. 5 or 10 mg) and polyol (i.e. 250 or 500 mg) in the chewing gum type G-R, S-DD, EE-NN and OO-WW, respectively.

The chewing gum particles containing gum base are prepared as described above in Example 1. The manufacturing of cetirizine and/or polyol in or between the chewing gum particles containing gum base is also performed as described in Example 1. Two chewing gum mixtures I and II are prepared comprising each cetirizine, polyol and other chewing gum ingredients. As outlines in below tables 2.1, 2.3, 2.5 and 2.7, within these mixture the cetirizine and polyol may be located either in or between the chewing gum particles containing gum base

Chewing gum mixture I is passed to a standard tablet pressing machine comprising dosing apparatus (e.g. P 3200 C, available from Fette GmbH, Germany) and compressed to form a first compressed module. Subsequent, 2 g of chewing gum mixture II is filed into the tablet pressing machine and compressed onto the first module to form a chewing gum tablet having two compressed modules. However, in some chewing gum tablets (i.e. chewing gum J, N, R, V, Z, DD, HH and OO-WW) tablet material (i.e. gum free module) is used instead of chewing gum particles comprising gum base. Examples of such tablet materials are described above.

**Table 2.1 Location of cetirizine and a polyol in a two-module compressed chewing gum tablet type G-R, wherein the first module comprises chewing gum particles containing gum base and cetirizine, and the second module comprises chewing gum particles containing gum base a polyol or tablet material and a polyol**

| **Chewing gum** | **Cetirizine location** | **Polyol location** |
|---|---|---|
| G | In the particles of first module | Between the particles of the second module |
| H | In the particles of first module | In the particles of the second module |
| I | In the particles of first module | Both in and between the particles of the second module |
| J | In the particles of first module | In a gum free module |
| K | Between the particles of the first module | Between the particles of the second module |
| L | Between the particles of the first module | In the particles of the second module |
| M | Between the particles of the first module | Both in and between the particles of the second module |
| N | Between the particles of the first module | In a gum free module |
| O | Both in and between the particles of the first module | Between the particles of the second module |
| P | Both in and between the particles of the first module | In the particles of the second module |
| Q | Both in and between the particles of the first module | Both in and between the particles of the second module |
| R | Both in and between the particles of the first module | In a gum free module |

**Table 2.2 Concentration of cetirizine and polyol in two-module compressed chewing gum tablet type G-R**

| **No.** | **Chewing** | **Conc. cetirizine** | **Conc. polyol** |
|---|---|---|---|
| 25 | G | 5 | 250 |
| 26 | G | 5 | 500 |
| 27 | G | 10 | 250 |
| 28 | G | 10 | 500 |
| 29 | H | 5 | 250 |
| 30 | H | 5 | 500 |
| 31 | H | 10 | 250 |
| 32 | H | 10 | 500 |
| 33 | I | 5 | 250 |
| 34 | I | 5 | 500 |
| 35 | I | 10 | 250 |
| 36 | I | 10 | 500 |
| 37 | J | 5 | 250 |
| 38 | J | 5 | 500 |
| 39 | J | 10 | 250 |
| 40 | J | 10 | 500 |
| 41 | K | 5 | 250 |
| 42 | K | 5 | 500 |
| 43 | K | 10 | 250 |
| 44 | K | 10 | 500 |
| 45 | L | 5 | 250 |
| 46 | L | 5 | 500 |
| 47 | L | 10 | 250 |
| 48 | L | 10 | 500 |
| 49 | M | 5 | 250 |
| 50 | M | 5 | 500 |
| 51 | M | 10 | 250 |
| 52 | M | 10 | 500 |
| 53 | N | 5 | 250 |
| 54 | N | 5 | 500 |
| 55 | N | 10 | 250 |
| 56 | N | 10 | 500 |
| 57 | O | 5 | 250 |
| 58 | O | 5 | 500 |
| 59 | O | 10 | 250 |
| 60 | O | 10 | 500 |
| 61 | P | 5 | 250 |
| 62 | P | 5 | 500 |
| 63 | P | 10 | 250 |
| 64 | P | 10 | 500 |
| 65 | Q | 5 | 250 |
| 66 | Q | 5 | 500 |
| 67 | Q | 10 | 250 |
| 68 | Q | 10 | 500 |
| 69 | R | 5 | 250 |
| 70 | R | 5 | 500 |
| 71 | R | 10 | 250 |
| 72 | R | 10 | 500 |

**Table 2.3 Location of cetirizine and a polyol in a two-module compressed chewing gum tablet types S-DD, wherein the first module comprises chewing gum particles containing gum base and a polyol, and the second module comprises chewing gum particles containing gum base and cetirizine or tablet material and cetirizine**

| **Chewing gum** | **Cetirizine location** | **Polyol location** |
|---|---|---|
| S | Between the particles of the second module | In the particles of first module |
| T | In the particles of the second module | In the particles of first module |
| U | Both in and between the particles of the second module | In the particles of first module |
| V | In a gum free module | In the particles of first module |
| W | Between the particles of the second module | Between the particles of the first module |
| X | In the particles of the second module | Between the particles of the first module |
| Y | Both in and between the particles of the second module | Between the particles of the first module |
| Z | In a gum free module | Between the particles of the first module |
| AA | Between the particles of the second module | Both in and between the particles of the first module |
| BB | In the particles of the second module | Both in and between the particles of the first module |
| CC | Both in and between the particles of the second module | Both in and between the particles of the first module |
| DD | In a gum free module | Both in and between the particles of the first module |

**Table 2.4 Concentration of cetirizine and polyol in two module compressed chewing gum tablet type S-DD.**

| **No.** | **Chewing gum** | **Conc. cetirizine mg** | **Conc. polyol mg** |
|---|---|---|---|
| 73 | S | 5 | 250 |
| 74 | S | 5 | 500 |
| 75 | S | 10 | 250 |
| 76 | S | 10 | 500 |
| 77 | T | 5 | 250 |
| 78 | T | 5 | 500 |
| 79 | T | 10 | 250 |
| 80 | T | 10 | 500 |
| 81 | U | 5 | 250 |
| 82 | U | 5 | 500 |
| 83 | U | 10 | 250 |
| 84 | U | 10 | 500 |
| 85 | V | 5 | 250 |
| 86 | V | 5 | 500 |
| 87 | V | 10 | 250 |
| 88 | V | 10 | 500 |
| 89 | W | 5 | 250 |
| 90 | W | 5 | 500 |
| 91 | W | 10 | 250 |
| 92 | W | 10 | 500 |
| 93 | X | 5 | 250 |
| 94 | X | 5 | 500 |
| 95 | X | 10 | 250 |
| 96 | X | 10 | 500 |
| 97 | Y | 5 | 250 |
| 98 | Y | 5 | 500 |
| 99 | Y | 10 | 250 |
| 100 | Y | 10 | 500 |
| 101 | Z | 5 | 250 |
| 102 | Z | 5 | 500 |
| 103 | Z | 10 | 250 |
| 104 | Z | 10 | 500 |
| 105 | AA | 5 | 250 |
| 106 | AA | 5 | 500 |
| 107 | AA | 10 | 250 |
| 108 | AA | 10 | 500 |
| 109 | BB | 5 | 250 |
| 110 | B6 | 5 | 500 |
| 111 | BB | 10 | 250 |
| 112 | BB | 10 | 500 |
| 113 | CC | 5 | 250 |
| 114 | CC | 5 | 500 |
| 115 | CC | 10 | 250 |
| 116 | CC | 10 | 500 |
| 117 | DD | 5 | 250 |
| 118 | DD | 5 | 500 |
| 119 | DD | 10 | 250 |
| 120 | DD | 10 | 500 |

**Table 2.5 Location of cetirizine and a polyol in a two-module compressed chewing gum tablet type EE-NN, wherein the first module comprises chewing gum particles containing gum base and the second module comprises chewing gum particles containing gum base, cetirizine and a polyol, or tablet material, cetirizine and a polyol**

| **Chewing gum** | **Cetirizine location** | **Polyol location** |
|---|---|---|
| EE | Between the particles of the second module | In the particles of second module |
| FF | In the particles of the second module | In the particles of second module |
| GG | Both in and between the particles of the second module | In the particles of second module |
| HH | In a gum free module | In a gum free module |
| II | Between the particles of the second module | Between the particles of the second module |
| JJ | In the particles of the second module | Between the particles of the second module |
| KK | Both in and between the particles of the second module | Between the particles of the second module |
| LL | Between the particles of the second module | Both in and between the particles of the second module |
| MM | In the particles of the second module | Both in and between the particles of the second module |
| NN | Both in and between the particles of the second module | Both in and between the particles of the second module |

**Table 2.6 Concentration of cetirizine and polyol in two module compressed chewing gum tablet type EE-NN**

| **No.** | **Chewing gum** | **Conc. cetirizine mg** | **Conc. polyol mg** |
|---|---|---|---|
| 121 | EE | 5 | 250 |
| 122 | EE | 5 | 500 |
| 123 | EE | 10 | 250 |
| 124 | EE | 10 | 500 |
| 125 | FF | 5 | 250 |
| 126 | FF | 5 | 500 |
| 127 | FF | 10 | 250 |
| 128 | FF | 10 | 500 |
| 129 | GG | 5 | 250 |
| 130 | GG | 5 | 500 |
| 131 | GG | 10 | 250 |
| 132 | GG | 10 | 500 |
| 133 | HH | 5 | 250 |
| 134 | HH | 5 | 500 |
| 135 | HH | 10 | 250 |
| 136 | HH | 10 | 500 |
| 137 | II | 5 | 250 |
| | | | |
| 138 | II | 5 | 500 |
| 139 | II | 10 | 250 |
| 140 | II | 10 | 500 |
| 141 | JJ | 5 | 250 |
| 142 | JJ | 5 | 500 |
| 143 | JJ | 10 | 250 |
| 144 | JJ | 10 | 500 |
| 145 | KK | 5 | 250 |
| 146 | KK | 5 | 500 |
| 147 | KK | 10 | 250 |
| 148 | KK | 10 | 500 |
| 149 | LL | 5 | 250 |
| 150 | LL | 5 | 500 |
| 151 | LL | 10 | 250 |
| 152 | LL | 10 | 500 |
| 153 | MM | 5 | 250 |
| 154 | MM | 5 | 500 |
| 155 | MM | 10 | 250 |
| 156 | MM | 10 | 500 |
| 157 | NN | 5 | 250 |
| 158 | NN | 5 | 500 |
| 159 | NN | 10 | 250 |
| 160 | NN | 10 | 500 |

**Table 2.7 Location of cetirizine and a polyol in a two-module compressed chewing gum tablet type OO-WW, wherein the first module comprises chewing gum particles containing gum base, cetirizine and a polyol and the second module comprises tablet material**

| **Chewing gum** | **Cetirizine location** | **Polyol location** |
|---|---|---|
| OO | Between the particles of the first module | In the particles of first module |
| PP | In the particles of the first module | In the particles of first module |
| QQ | Both in and between the particles of the first module | In the particles of first module |
| RR | Between the particles of the | Between the particles of the |

| | first module | first module |
|---|---|---|
| SS | In the particles of the first module | Between the particles of the first module |
| TT | Both in and between the particles of the first module | Between the particles of the first module |
| UU | Between the particles of the first module | Both in and between the particles of the first module |
| VV | In the particles of the first module | Both in and between the particles of the first module |
| WW | Both in and between the particles of the first module | Both in and between the particles of the first module |

**Table 2.8 Concentration of cetirizine and polyol in two-module compressed chewing gum tablet OO-WW**

| **No.** | **Chewing gum** | **Conc. cetirizine** | **Conc. polyol** |
|---|---|---|---|
| 161 | OO | 5 | 250 |
| 162 | OO | 5 | 500 |
| 163 | OO | 10 | 250 |
| 164 | OO | 10 | 500 |
| 165 | PP | 5 | 250 |
| 166 | PP | 5 | 500 |
| 167 | PP | 10 | 250 |
| 168 | PP | 10 | 500 |
| 169 | QQ | 5 | 250 |
| 170 | QQ | 5 | 500 |
| 171 | QQ | 10 | 250 |
| 172 | QQ | 10 | 500 |
| 173 | RR | 5 | 250 |
| 174 | RR | 5 | 500 |
| 175 | RR | 10 | 250 |
| 176 | RR | 10 | 500 |
| 177 | SS | 5 | 250 |
| 178 | SS | 5 | 500 |
| 179 | SS | 10 | 250 |
| 180 | SS | 10 | 500 |
| 181 | TT | 5 | 250 |
| 182 | TT | 5 | 500 |
| 183 | TT | 10 | 250 |
| 184 | TT | 10 | 500 |
| 185 | UU | 5 | 250 |
| 186 | UU | 5 | 500 |
| 187 | UU | 10 | 250 |
| 188 | UU | 10 | 500 |
| 189 | VV | 5 | 250 |
| 190 | VV | 5 | 500 |
| 191 | VV | 10 | 250 |
| 192 | VV | 10 | 500 |
| 193 | WW | 5 | 250 |
| 194 | WW | 5 | 500 |
| 195 | WW | 10 | 250 |
| 196 | WW | 10 | 500 |

### example 3

### Compressed chewing gum tablet having two compressed modules

A compressed chewing gum tablets having two compressed modules comprising cetirizine and polyol (taste-masking agent) was prepared.

### The first module (layer) contained

| | |
|---|---|
| Gum base (with antioxidant BHT=700 ppm) | 400 gram |
| Isomalt (for direct compression) | 527 gram |
| Twin Sweet | 3 gram |
| Grapefruit flavour | 60 gram |
| Magnesium stearate | 10 gram |

### The second module (layer) contained

| | |
|---|---|
| Cetirizine | 10 gram |
| Magnesium stearate | 0.8 gram |
| Grapefruit flavour | 8.0 gram |
| Sorbitol | 310.8 gram |
| Saccharin sodium | 0.4 gram |

The ingredients for each module were mixed dry in a conventional dry mixer and formed into a tablet in a two station tablet machine as described in Example 1.

The mixtures gave a total of 1000 tablets where each tablet is made op of module 1 = 1000 mg and module 2 = 400 mg. The content of Cetirizine is 10 mg per chewing gum piece. If a chewing gum with 5 mg cetirizine is desired, 5 gram of cetirizine is added in the portion for the second module and the sorbitol content is adjusted to 315.8 gram.

## Claims

1. A compressed chewing gum tablet comprising at least one active compound selected from 2-[4-(diphenylmethyl)-1-piperazine] derivatives having the general formula I: 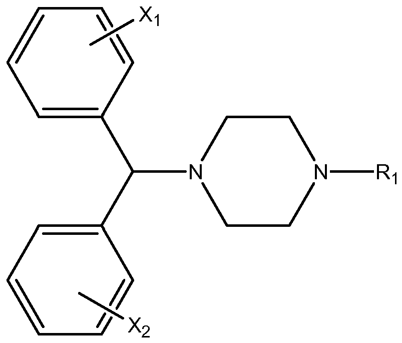 wherein
R₁ is selected from the group consisting of -CH₂CH₂-O-CH₂-R₂, -CH₂CH=CH-Ar₁, -CH₂-Ar₂ and 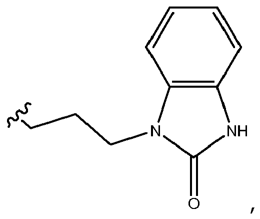
R₂ may be selected from the group consisting of a -CH₂OH group, a -COOH group and a - CONH₂ group;
Ar₁ and Ar₂ are independently an aromatic or heteroaromatic ring with 5 or 6 atoms in the ring, said heteroaromatic ring having 1, 2 or 3 heteroatoms selected from the group consisting of nitrogen, oxygen and sulfur, said ring being unsubstituted or substituted with C₁₋₄ alkyl, preferably methyl or tertiary butyl, Ar₁ and Ar₂ preferably being unsubstituted phenyl or phenyl substituted with C₁₋₄ alkyl, preferably methyl or tertiary butyl, and
X₁ and X₂ may independently be selected from the group consisting of a hydrogen atom, a halogen atom, a straight-chain or branched C₁-C₄ alkoxy group or a trifluoromethyl group; as well as pharmaceutically acceptable salts, geometrical isomers, enantiomers, diastereomers and mixtures thereof;
a taste-masking agent and a first compressed module comprising compressed chewing gum particles containing gum base;
wherein the weight ratio between the taste-masking agent and the compound according to formula I is at least 5: 1.

2. The compressed chewing gum tablet according to claim 1, wherein the first compressed module is on top of a second compressed module.

3. The compressed chewing gum tablet according to claim 1 or 2, wherein
a) the first compressed module is on top of a second compressed module, said second compressed module comprises an active compound according to formula I and a taste-masking agent, and the weight ratio between the taste-masking agent of the second compressed module and the compound according to formula I of the second compressed module is at least 5: 1, or
b) the weight ratio between the taste-masking agent of the first compressed module and the compound according to formula I of the first compressed module is at least 5:1.

4. The compressed chewing gum tablet according to any of the claims 2-3 comprising a first and a second compressed module, wherein the first compressed module comprises compressed chewing gum particles containing gum base, an active compound according to formula I, and a taste-masking agent.

5. The compressed chewing gum tablet according to any of the claims 2-4, comprising a first and a second compressed module, wherein the first compressed module comprises compressed chewing gum particles containing gum base, and the second compressed module comprises an active compound according to formula I and a taste-masking agent.

6. The compressed chewing gum tablet according to any of the preceding claims, wherein the weight ratio between the taste-masking agent of the first compressed module and the compound according to formula I of the first compressed module is at least 5:1 and wherein the compound according to formula I and the taste-masking agent are located between the compressed chewing gum particles containing gum base of the first compressed module.

7. The compressed chewing gum tablet according to any of the preceding claims, wherein the chewing gum particles comprise an amount of high intensity sweetener.

8. The compressed chewing gum tablet according to any of the preceding claims, wherein the compound according to formula I and the taste-masking agent are located between the compressed chewing gum particles containing gum base of the first and/or second compressed module.

9. The compressed chewing gum tablet of any of the preceding claims, wherein the taste-masking agent comprises a polyol sweetener.

10. The compressed chewing gum tablet of any of the preceding claims, wherein the taste-masking agent comprising a high intensity sweetener or a flavour.

11. The compressed chewing gum tablet according to claim 8 or 9, wherein the taste-masking agent comprises high intensity sweetener in an amount in the range of 0.01-5% by weight of the taste masking agent.

12. The compressed chewing gum tablet according to any of the preceding claims, wherein the active compound according to formula I is selected from the group consisting of buclizine, cetirizine, chlorcyclizine, cinnarizine, cyclizine, hydroxyzine, levocetirizine, meclozine, efletirizine and oxatomide, as well as any pharmaceutically acceptable salts, geometrical isomers, enantiomers, diastereomers and mixtures thereof.

13. The compressed chewing gum tablet according to any of the preceding claims, wherein the active compound according to formula I is cetirizine or a salt thereof, preferably cetirizine dihydrochloride.

14. The compressed chewing gum tablet according to any of the preceding claims, further comprising one or more alkalizing agent(s).

15. A method of preparing a compressed chewing gum tablet according to claim 1 comprising one compressed module, the method comprising the steps of:
a) providing a portion comprising an active compound according to formula I, a portion comprising taste-masking agent, and chewing gum particles containing gum base;
b) optionally providing one or more further chewing gum ingredients;
c) dosing the portion comprising the compound according to formula I, the portion comprising taste-masking agent, and the chewing gum particles containing gum base, and optionally the one or more further chewing gum ingredients; and
d) compressing a) and b) after dosing, to obtain a first compressed module.

16. The method according to claim 15, furthermore comprising the steps of
e) providing a portion comprising tablet material;
f) contacting the first compressed module with the portion of step e); and
g) compressing e) and the first compressed module to obtain a coherent compressed chewing gum tablet comprising a first and a second compressed module.

17. A method of preparing a compressed chewing gum tablet according to claim 1 comprising two compressed modules, the method comprising the steps of:
a) providing chewing gum particles containing gum base and optionally portion(s) comprising one or more chewing gum ingredients;
b) providing a portion comprising an active compound according to formula I and a portion comprising a taste-masking agent;
c) compressing a) to obtain a first compressed module;
d) contacting the first compressed module with b); and
e) compressing b) and the first compressed module, to obtain a coherent compressed chewing gum tablet comprising a first compressed module and a second compressed module.

18. A method of preparing a compressed chewing gum tablet according to claim 1 comprising two compressed modules, the method comprising the steps of:
a) providing chewing gum particles containing gum base and a portion comprising an active compound according to formula I, and optionally portion(s) comprising one or more chewing gum ingredients;
b) providing a portion comprising taste-masking agent;
c) compressing a) to obtain a first compressed module;
d) contacting the first compressed module with b); and
e) compressing b) and the first compressed module, to obtain a coherent compressed chewing gum tablet comprising a first compressed module and a second compressed module.
